# EUROPEAN PATENT SPECIFICATION

(11) **EP 2 610 347 B1**
(45) Date of publication and mention of the grant of the patent: **15.04.2015**
(21) Application number: 13159600.9
(22) Date of filing: 29.04.2008
(51) Int. Cl.: C12P 19/34, C12Q 1/68

(54) **Methods of determining the prognosis of a subject with pancreatic cancer**
Verfahren zur Bestimmung der Prognose eines Subjekts mit Bauchspeicheldrüsenkrebs
Procédés de détermination du pronostic d'un sujet avec un cancer du pancréas

(30) Priority: 30.04.2007 US 926933 P
(43) Date of publication of application: 03.07.2013
(62) Divisional of application: 12165636.7
(73) Proprietor: The Ohio State University Research Foundation, Columbus, OH 43201 (US)
(72) Inventor: Croce, Carlo, Columbus, OH 43221 (US)
(74) Representative: Turner, Craig Robert

(56) References cited:
- EP-A1- 2 487 240
- WO-A2-2005/078139
- ROLDO CLAUDIA ET AL: "MICRORNA EXPRESSION ABNORMALITIES IN PANCREATIC ENDOCRINE AND ACINAR TUMORS ARE ASSOCIATED WITH DISTINCTIVE PATHOLOGIC FEATURES AND CLINICAL BEHAVIOR", JOURNAL OF CLINICAL ONCOLOGY, AMERICAN SOCIETY OF CLINICAL ONCOLOGY, US LNKD- DOI:10.1200/JCO.2005.05.5194, vol. 24, no. 29, 1 October 2006 (2006-10-01), pages 4677-4684, XP008078235, ISSN: 0732-183X
- VOLINIA STEFANO ET AL: "A microRNA expression signature of human solid tumors defines cancer gene targets", PROCEEDINGS OF THE NATIONAL ACADEMY OF SCIENCES OF THE UNITED STATES OF AMERICA, vol. 103, no. 7, February 2006 (2006-02), pages 2257-2261, XP002578086, ISSN: 0027-8424
- LEE EUN JON ET AL: "Expression profiling identifies microRNA signature in pancreatic cancer", INTERNATIONAL JOURNAL OF CANCER,, vol. 120, no. 5, 1 March 2007 (2007-03-01) , pages 1046-1054, XP002569593,
- MENG ET AL: "Involvement of Human Micro-RNA in Growth and Response to Chemotherapy in Human Cholangiocarcinoma Cell Lines", GASTROENTEROLOGY, ELSEVIER, PHILADELPHIA, PA LNKD- DOI:10.1053/J.GASTRO.2006.02.057, vol. 130, no. 7, 1 June 2006 (2006-06-01), pages 2113-2129, XP005475314, ISSN: 0016-5085
- RONGE R: "Mikro-RNA: Wegweisend für Diagnose und Prognose", ZEITSCHRIFT FUER GASTROENTEROLOGIE, GEORG THIEME VERLAG, DE, vol. 45, no. 8, 1 January 2007 (2007-01-01), page 682, XP008152347, ISSN: 0044-2771
- XIANGYU KONG ET AL: "Detection of Differentially Expressed microRNAs in Serum of Pancreatic Ductal Adenocarcinoma Patients: miR-196a Could Be a Potential Marker for Poor Prognosis", DIGESTIVE DISEASES AND SCIENCES, KLUWER ACADEMIC PUBLISHERS-PLENUM PUBLISHERS, NE, vol. 56, no. 2, 8 July 2010 (2010-07-08), pages 602-609, XP019877637, ISSN: 1573-2568, DOI: 10.1007/S10620-010-1285-3
- BLOOMSTON MARK ET AL: "MicroRNA expression patterns to differentiate pancreatic adenocarcinoma fromnormal pancreas and chronic pancreatitis", JAMA THE JOURNAL OF THE AMERICAN MEDICAL ASSOCIATION, AMERICAN MEDICAL ASSOCIATION, US, vol. 297, no. 17, 2 May 2007 (2007-05-02), pages 1901-1908, XP009092914, ISSN: 0098-7484, DOI: 10.1001/JAMA.297.17.1901

### SEQUENCE LISTING

<110> OHIO STATE UNIVERSITY
<120> METHODS FOR DIFFERENTIATING PANCREATIC CANCER FROM NORMAL PANCREATIC FUNCTION AND/OR CHRONIC PANCREATITIS
<130> 53-29071
<140>
   <141>
<150> 60/926,933 <151> 2007-04-30
<160> 60
<170> PatentIn version 3.3
<210> 1
   <211> 23
   <212> RNA
   <213> Homo sapiens
<400> 1
   agcuacauug ucugcugggu uuc 23
<210> 2
   <211> 23
   <212> RNA
   <213> Homo sapiens
<400> 2
   aacauucaac gcugucggug agu 23
<210> 3
   <211> 23
   <212> RNA
   <213> Homo sapiens
<400> 3
   uuaaugcuaa ucgugauagg ggu 23
<210> 4
   <211> 22
   <212> RNA
   <213> Homo sapiens
<400> 4
   cugugcgugu gacagcggcu ga 22
<210> 5
   <211> 23
   <212> RNA
   <213> Homo sapiens
<400> 5
   aacauucaac gcugucggug agu 23
<210> 6
   <211> 23
   <212> RNA
   <213> Homo sapiens
<400> 6
   aacauucauu gcugucggug ggu 23
<210> 7
   <211> 23
   <212> RNA
   <213> Homo sapiens
<400> 7
   aacauucauu gcugucggug ggu 23
<210> 8
   <211> 21
   <212> RNA
   <213> Homo sapiens
<400> 8
   agcuacaucu ggcuacuggg u 21
<210> 9
   <211> 22
   <212> RNA
   <213> Homo sapiens
<400> 9
   uagcuuauca gacugauguu ga 22
<210> 10
   <211> 22
   <212> RNA
   <213> Homo sapiens
<400> 10
   aacauucaac cugucgguga gu 22
<210> 11
   <211> 23
   <212> RNA
   <213> Homo sapiens
<400> 11
   aacauucauu guugucggug ggu 23
<210> 12
   <211> 21
   <212> RNA
   <213> Homo sapiens
<400> 12
   ccacaccgua ucugacacuu u 21
<210> 13
   <211> 22
   <212> RNA
   <213> Homo sapiens
<400> 13
   ugucaguuug ucaaauaccc ca 22
<210> 14
   <211> 22
   <212> RNA
   <213> Homo sapiens
<400> 14
   aacccguaga uccgaacuug ug 22
<210> 15
   <211> 24
   <212> RNA
   <213> Homo sapiens
<400> 15
   ucccugagac ccuuuaaccu guga 24
<210> 16
   <211> 22
   <212> RNA
   <213> Homo sapiens
<400> 16
   ucccugagac ccuaacuugu ga 22
<210> 17
   <211> 21
   <212> RNA
   <213> Homo sapiens
<400> 17
   ugagaugaag cacuguagcu c 21
<210> 18
   <211> 23
   <212> RNA
   <213> Homo sapiens
<400> 18
   uacccuguag auccgaauuu gug 23
<210> 19
   <211> 22
   <212> RNA
   <213> Homo sapiens
<400> 19
   ugagaacuga auuccauggg uu 22
<210> 20
   <211> 22
   <212> RNA
   <213> Homo sapiens
<400> 20
   aacccguaga uccgaucuug ug 22
<210> 21
   <211> 23
   <212> RNA
   <213> Homo sapiens
<400> 21
   cccaguguuc agacuaccug uuc 23
<210> 22
   <211> 23
   <212> RNA
   <213> Homo sapiens
<400> 22
   cccaguguuc agacuaccug uuc 23
<210> 23
   <211> 23
   <212> RNA
   <213> Homo sapiens
<400> 23
   cccaguguuu agacuaucug uuc 23
<210> 24
   <211> 23
   <212> RNA
   <213> Homo sapiens
<400> 24
   uacccuguag aaccgaauuu gug 23
<210> 25
   <211> 23
   <212> RNA
   <213> Homo sapiens
<400> 25
   agcagcauug uacagggcua uca 23
<210> 26
   <211> 23
   <212> RNA
   <213> Homo sapiens
<400> 26
   agcagcauug uacagggcua uga 23
<210> 27
   <211> 22
   <212> RNA
   <213> Homo sapiens
<400> 27
   uccuucauuc caccggaguc ug 22
<210> 28
   <211> 21
   <212> RNA
   <213> Homo sapiens
<400> 28
   aucacauugc cagggauuac c 21
<210> 29
   <211> 21
   <212> RNA
   <213> Homo sapiens
<400> 29
   aucacauugc cagggauuuc c 21
<210> 30
   <211> 22
   <212> RNA
   <213> Homo sapiens
<400> 30
   ucagugcacu acagaacuuu gu 22
<210> 31
   <211> 22
   <212> RNA
   <213> Homo sapiens
<400> 31
   ucagugcauc acagaacuuu gu 22
<210> 32
   <211> 22
   <212> RNA
   <213> Homo sapiens
<400> 32
   uuuguucguu cggcucgcgu ga 22
<210> 33
   <211> 23
   <212> RNA
   <213> Homo sapiens
<400> 33
   uuuggcacua gcacauuuuu gcu 23
<210> 34
   <211> 22
   <212> RNA
   <213> Homo sapiens
<400> 34
   uggcaguguc uuagcugguu gu 22
<210> 35
   <211> 21
   <212> RNA
   <213> Homo sapiens
<400> 35
   cagcagcaca cugugguuug u 21
<210> 36
   <211> 22
   <212> RNA
   <213> Homo sapiens
<400> 36
   gugaaauguu uaggaccacu ag 22
<210> 37
   <211> 23
   <212> RNA
   <213> Homo sapiens
<400> 37
   agguuguccg uggugaguuc gca 23
<210> 38
   <211> 23
   <212> RNA
   <213> Homo sapiens
<400> 38
   caaagugcug uucgugcagg uag 23
<210> 39
   <211> 22
   <212> RNA
   <213> Homo sapiens
<400> 39
   uauugcacuu gucccggccu gu 22
<210> 40
   <211> 22
   <212> RNA
   <213> Homo sapiens
<400> 40
   ugaaacauac acgggaaacc uc 22
<210> 41
   <211> 22
   <212> RNA
   <213> Homo sapiens
<400> 41
   aagacgggag gaaagaaggg ag 22
<210> 42
   <211> 23
   <212> RNA
   <213> Homo sapiens
<400> 42
   ucccuguccu ccaggagcuc acg 23
<210> 43
   <211> 21
   <212> RNA
   <213> Homo sapiens
<400> 43
   uugugcuuga ucuaaccaug u 21
<210> 44
   <211> 22
   <212> RNA
   <213> Homo sapiens
<400> 44
   gaauguugcu cggugaaccc cu 22
<210> 45
   <211> 22
   <212> RNA
   <213> Homo sapiens
<400> 45
   agaucagaag gugauugugg cu 22
<210> 46
   <211> 22
   <212> RNA
   <213> Homo sapiens
<400> 46
   uucaccaccu ucuccaccca gc 22
<210> 47
   <211> 22
   <212> RNA
   <213> Homo sapiens
<400> 47
   uguaacagca acuccaugug ga 22
<210> 48
   <211> 22
   <212> RNA
   <213> Homo sapiens
<400> 48
   gguccagagg ggagauaggu uc 22
<210> 49
   <211> 23
   <212> RNA
   <213> Homo sapiens
<400> 49
   uggaagacua gugauuuugu ugu 23
<210> 50
   <211> 21
   <212> RNA
   <213> Homo sapiens
<400> 50
   ucacagugaa ccggucucuu u 21
<210> 51
   <211> 21
   <212> RNA
   <213> Homo sapiens
<400> 51
   uagcagcaca gaaauauugg c 21
<210> 52
   <211> 22
   <212> RNA
   <213> Homo sapiens
<400> 52
   uagguaguuu cauguuguug gg 22
<210> 53
   <211> 22
   <212> RNA
   <213> Homo sapiens
<400> 53
   aacuguuugc agaggaaacu ga 22
<210> 54
   <211> 23
   <212> RNA
   <213> Homo sapiens
<400> 54
   ucaaaugcuc agacuccugu ggu 23
<210> 55
   <211> 22
   <212> RNA
   <213> Homo sapiens
<400> 55
   cugaagcuca gagggcucug au 22
<210> 56
   <211> 20
   <212> RNA
   <213> Homo sapiens
<400> 56
   cugcaaaggg aagcccuuuc 20
<210> 57
   <211> 22
   <212> RNA
   <213> Homo sapiens
<400> 57
   ucgugucuug uguugcagcc gg 22
<210> 58
   <211> 22
   <212> RNA
   <213> Homo sapiens
<400> 58
   uguaaacauc cucgacugga ag 22
<210> 59
   <211> 22
   <212> RNA
   <213> Homo sapiens
<400> 59
   ucccugagac ccuaacuugu ga 22
<210> 60
   <211> 22
   <212> RNA
   <213> Homo sapiens
<400> 60
   ucguaccgug aguaauaaug cg 22

## Description

### BACKGROUND

Pancreatic cancer is a lethal disease with annual mortality nearly equaling incidence of approximately 33,000 in the United States.¹ While stage migration is partly to blame for the poor survival, the biology of ductal adenocarcinoma of the pancreas is one of aggressive local invasion, early metastasis, and resistance to chemotherapy and radiation. Known genetic mutations including *TP53, KRAS, CDKN2A,* and *SMAD4²* are important in pancreatic cancer but individually do not account for its aggressive behavior.

MicroRNAs (miRNAs) are small noncoding RNAs that are cleaved from 70- to 100-nucleotide hairpin pre-miRNA precursors in the cytoplasm by RNase III Dicer into their mature form of 19- to 25-nucleotides.³ Single-stranded miRNAs bind mRNAs of potentially hundreds of genes at the 3' untranslated region with perfect or near-perfect complementarity resulting in degradation or inhibition of the target mRNA, respectively. In humans, aberrant expression of miRNAs contributes to carcinogenesis by promoting the expression of proto-oncogenes or inhibiting the expression of tumor suppressor genes.⁴ Such "oncomiRs" have been demonstrated in a variety of hematologic and solid malignancies.⁵⁻⁷

Identification of microRNAs that are differentially-expressed in pancreatic cancer cells may help pinpoint specific miRNAs that are involved in pancreatic cancer (e.g., pancreatic endocrine tumors, acinar carcinomas). Furthermore, the identification of putative targets of these miRNAs may help to unravel their pathogenic role. Described herein are methods and compositions for the diagnosis, prognosis and treatment of pancreatic cancer.

MiRNA markers for the prognosis of pancreatic cancer are known in the field, see for example Roldo et al., Journal of Clinical Oncology, vol. 24, no. 29, pages 4677-4684, October 2006; and Ronge, Zeitschrift für Gastroenterologie, vol 45, no. 8, page 682, January 2007.

### SUMMARY

The identification of specific miRNAs associated with altered expression levels in pancreatic cancer cells is disclosed herein.

Also disclosed herein is a method of diagnosing whether a subject has, or is at risk for developing, pancreatic cancer, comprising measuring the level of at least one miR gene product in a test sample from said subject, wherein an alteration in the level of the miR gene product in the test sample, relative to the level of a corresponding miR gene product in a control sample, is indicative of the subject either having, or being at risk for developing, pancreatic cancer.

Also disclosed herein is a method of differentiating pancreatic cancer from at least one of normal pancreatic tissue and chronic pancreatitis in a human patient, comprising: detecting the level of expression in a tissue sample at least one miR gene product from at least one of Tables 1a, 1b, 1c, 2a, 2b, 2c and 3; where differential expression is indicative of pancreatic cancer rather than normal pancreas or chronic pancreatitis.

Also disclosed herein is a method of determining the prognosis of a subject with pancreatic cancer, comprising measuring the level of at least one miR gene product in a test sample from said subject, wherein: the miR gene product is associated with an adverse prognosis in pancreatic cancer; and an alteration in the level of the at least one miR gene product in the pancreatic test sample, relative to the level of a corresponding miR gene product in a control sample, is indicative of an adverse prognosis.

According to a first aspect of the present invention, there is provided a method of determining the prognosis of a subject with pancreatic cancer, comprising measuring the level of at least miR-196a-2 in a test sample from said subject, wherein a decrease in the level of the at least one miR gene product, relative to the level of the corresponding miR gene product in a control sample, is indicative of an adverse prognosis. In one embodiment, the level of miR-196a-2 and miR-219 is measured.

According to a second aspect of the present invention, there is provided the use of a kit comprising one or more solid supports having attached thereto one or more oligodeoxynucleotides complementary to miR-196a-2 to determine the prognosis of a subject with pancreatic cancer by measuring the level of at least miR-196a-2 in a test sample from said subject, wherein a decrease in the level of the at least one miR gene product, relative to the level of the corresponding miR gene product in a control sample, is indicative of an adverse prognosis. In one embodiment, the level of miR-196a-2 and miR-219 is measured.

One method disclosed herein includes diagnosing whether a subject has, or is at risk for developing, pancreatic cancer. In a particular aspect, the level of at least one miR gene product in a test sample from the subject is compared to the level of a corresponding miR gene product in a control sample. An alteration (e.g., an increase, a decrease) in the level of the miR gene product in the test sample, relative to the level of a corresponding miR gene product in the control sample, is indicative of the subject either having, or being at risk for developing, pancreatic cancer.

The pancreatic cancer that is diagnosed may be a pancreatic exocrine tumor (e.g., an adenocarcinoma). The method may be used to distinguish a pancreatic endocrine tumor (PET) from a pancreatic exocrine tumor (e.g., an adenocarcinoma). The diagnostic method may be used to diagnose any type of pancreatic cancer.

Also disclosed is a method of diagnosing whether a subject has, or is at risk for developing, pancreatic adenocarcinoma. In this method, the level of at least one miR gene product in a test sample from the subject is compared to the level of a corresponding miR gene product in a control sample. An alteration (e.g., an increase, a decrease) in the level of the miR gene product in the test sample, relative to the level of a corresponding miR gene product in a control sample, is indicative of the subject either having, or being at risk for developing, pancreatic adenocarcinoma. The level of the at least one miR gene product in the test sample may be greater than the level of the corresponding miR gene product in the control sample.

Also disclosed is a method of diagnosing the type of pancreatic cancer that a subject has. In this method, the level of at least one miR gene product in a test sample from the subject is compared to the level of a corresponding miR gene product in a control sample. An alteration (e.g., an increase, a decrease) in the level of the miR gene product in the test sample, relative to the level of a corresponding miR gene product in a control sample, is indicative of the type of pancreatic cancer.

The type of pancreatic cancer that is diagnosed may be selected from the group consisting of adenocarcinoma. The level of the at least one miR gene product in the test sample may be greater than the level of the corresponding miR gene product in the control sample.

The level of the at least one miR gene product in the test sample may be greater than the level of the corresponding miR gene product in the control sample.

Also disclosed is a method of determining the prognosis of a subject with pancreatic cancer. In this method, the level of at least one miR gene product, which is associated with an adverse prognosis in pancreatic cancer, is measured in a test sample (e.g., a pancreatic cancer sample) from the subject. An alteration (e.g., an increase, a decrease) in the level of the miR gene product in the test sample, relative to the level of a corresponding miR gene product in a control sample, is indicative of an adverse prognosis. The level of the at least one miR gene product in the test sample may be greater than the level of the corresponding miR gene product in a control sample. The at least one miR gene product that is measured may be miR-196a-2 **[SEQ ID NO: 52]**. The pancreatic cancer may be associated with metastasis and/or a high proliferation index.

Also disclosed is a method of determining whether a pancreatic cancer in a subject is metastatic. In this method, the level of at least one miR gene product is measured in a test sample (e.g., a pancreatic cancer sample) from the subject. An alteration (e.g., an increase, a decrease) in the level of the miR gene product in the test sample, relative to the level of a corresponding miR gene product in a control sample, is indicative of metastasis. The level of the at least one miR gene product in the test sample may be greater than the level of the corresponding miR gene product in the control sample.

Also disclosed is a method of determining whether a pancreatic cancer in a subject has a high proliferation index. In this method, the level of at least one miR gene product is measured in a test sample (e.g., a pancreatic cancer sample) from the subject. An alteration (e.g., an increase, a decrease) in the level of the miR gene product in the test sample, relative to the level of a corresponding miR gene product in a control sample, is indicative of a high proliferation index. The level of the at least one miR gene product in the test sample may be greater than the level of the corresponding miR gene product in the control sample. The at least one miR gene product may be miR-196a-2 **[SEQ ID NO: 52]** or miR-219 **[SEQ ID NO: 64]**.

Also disclosed is a method of determining the prognosis of a subject with pancreatic cancer. In this method, the level of PDCD4 is measured in a test sample (e.g., a pancreatic cancer sample) from the subject. An alteration (e.g., an increase, a decrease) in the level of PDCD4 in the test sample, relative to the level of PDCD4 in a control sample, is indicative of an adverse prognosis. The level of PDCD4 in the test sample may be less than the level of PDCD4 in the control sample. The pancreatic cancer may be associated with metastasis and/or a high proliferation index.

The level of the at least one miR gene product can be measured using a variety of techniques that are well known to those of skill in the art (e.g., quantitative or semiquantitative RT-PCR, Northern blot analysis, solution hybridization detection). The level of at least one miR gene product may be measured by reverse transcribing RNA from a test sample obtained from the subject to provide a set of target oligodeoxynucleotides, hybridizing the target oligodeoxynucleotides to one or more miRNA-specific probe oligonucleotides (e.g., a microarray that comprises miRNA-specific probe oligonucleotides) to provide a hybridization profile for the test sample, and comparing the test sample hybridization profile to a hybridization profile generated from a control sample. An alteration in the signal of at least one miRNA in the test sample relative to the control sample is indicative of the subject either having, or being at risk for developing, pancreatic cancer. The signal of at least one miRNA may be upregulated, relative to the signal generated from the control sample. The signal of at least one miRNA may be downregulated, relative to the signal generated from the control sample. The microarray may comprise miRNA-specific probe oligonucleotides for a substantial portion of all known human miRNAs.

There is also disclosed methods of diagnosing whether a subject has, or is at risk for developing, a pancreatic cancer with an adverse prognosis. In one method, the level of at least one miR gene product, which is associated with an adverse prognosis in pancreatic cancer, is measured by reverse transcribing RNA from a test sample obtained from the subject to provide a set of target oligodeoxynucleotides. The target oligodeoxynucleotides are then hybridized to one or more miRNA-specific probe oligonucleotides (e.g., a microarray that comprises miRNA-specific probe oligonucleotides) to provide a hybridization profile for the test sample, and the test sample hybridization profile is compared to a hybridization profile generated from a control sample. An alteration in the signal of at least one miRNA in the test sample relative to the control sample is indicative of the subject either having, or being at risk for developing, a pancreatic cancer with an adverse prognosis.

There is also disclosed methods of treating pancreatic cancer in a subject, wherein at least one miR gene product is deregulated (e.g., downregulated, upregulated) in the cancer cells of the subject. When at least one isolated miR gene product is downregulated in the pancreatic cancer cells, the method comprises administering an effective amount of an isolated miR gene product, or an isolated variant or biologically-active fragment thereof, such that proliferation of cancer cells in the subject is inhibited.

When at least one isolated miR gene product is upregulated in the cancer cells, the method comprises administering to the subject an effective amount of at least one compound for inhibiting expression of the at least one miR gene product, such that proliferation of pancreatic cancer cells is inhibited.

The methods of treating pancreatic cancer in a subject may additionally comprise the step of first determining the amount of at least one miR gene product in pancreatic cancer cells from the subject, and comparing that level of the miR gene product to the level of a corresponding miR gene product in control cells. If expression of the miR gene product is deregulated (e.g., downregulated, upregulated) in pancreatic cancer cells, the methods further comprise altering the amount of the at least one miR gene product expressed in the pancreatic cancer cells. The amount of the miR gene product expressed in the cancer cells may be less than the amount of the miR gene product expressed in control cells, and an effective amount of the miR gene product, or an isolated variant or biologically-active fragment thereof, is administered to the subject. The amount of the miR gene product expressed in the cancer cells may be greater than the amount of the miR gene product expressed in control cells, and an effective amount of at least one compound for inhibiting expression of the at least one miR gene is administered to the subject. Suitable miRs and compounds that inhibit expression of miR genes include, for example, those described herein.

There is also disclosed pharmaceutical compositions for treating pancreatic cancer. The pharmaceutical compositions may comprise at least one isolated miR gene product, or an isolated variant or biologically-active fragment thereof, and a pharmaceutically-acceptable carrier. The at least one miR gene product may correspond to a miR gene product that has a decreased level of expression in pancreatic cancer cells relative to suitable control cells (i.e., it is downregulated).

The pharmaceutical compositions may comprise at least one miR expression-inhibition compound and a pharmaceutically-acceptable carrier. The at least one miR expression-inhibition compound may be specific for a miR gene product whose expression is greater in pancreatic cancer cells than control cells (i.e., it is upregulated).

There is also disclosed methods of identifying an anti-pancreatic cancer agent, comprising providing a test agent to a cell and measuring the level of at least one miR gene product in the cell. The method may comprise providing a test agent to a cell and measuring the level of at least one miR gene product associated with decreased expression levels in pancreatic cancer cells. An increase in the level of the miR gene product in the cell, relative to a suitable control cell, is indicative of the test agent being an anti-pancreatic cancer agent.

The method may comprise providing a test agent to a cell and measuring the level of at least one miR gene product associated with increased expression levels in pancreatic cancer cells. A decrease in the level of the miR gene product associated with increased expression levels in pancreatic cancer in the cell, relative to a suitable control cell, is indicative of the test agent being an anti-pancreatic cancer agent.

### BRIEF DESCRIPTION OF THE DRAWINGS

The invention can be more fully understood from the following detailed description, the drawings and the Sequence Descriptions that form a part of this application.

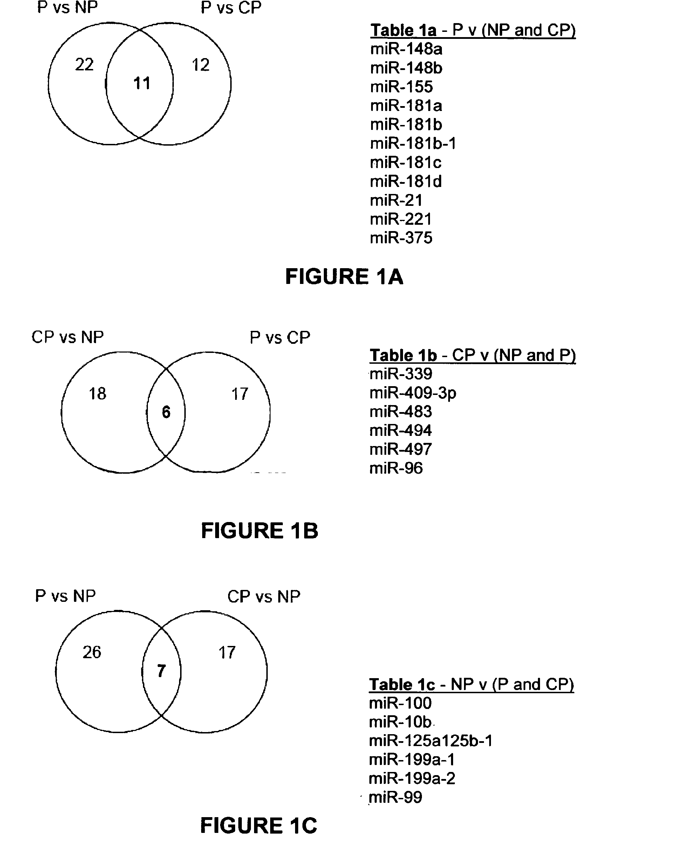

Figures 1A, 1B and 1C show a Venn diagram illustrating the relationships between sets of miRNAs found to be differentially expressed by pairwise comparisons between tissue types; and show Tables 1a, 1b and 1c listing the sets of miRNAs found to be differentially expressed by pairwise comparisons between tissue types.

Circles include the total number of differentially expressed miRNAs in the pairwise comparison indicated. Intersecting areas demonstrate the number of differentially expressed miRNAs in common between each comparison. The common miRNAs are listed for each set. NP, normal pancreas; P, pancreatic cancer; CP, chronic pancreatitis.

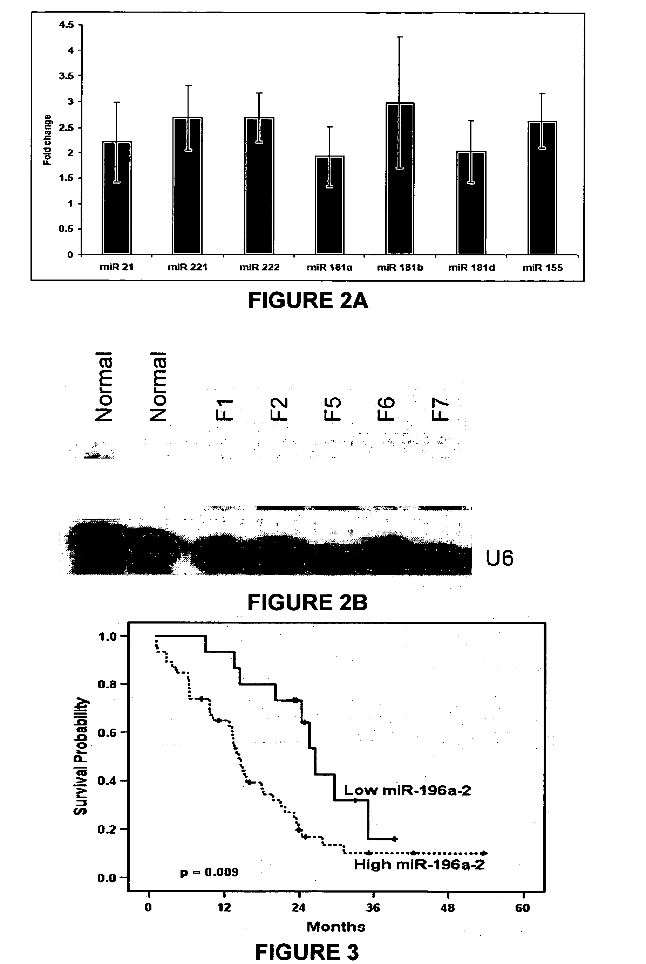

Figures 2A and 2B shows an analysis of differentially expressed microRNAs. Figure 2A shows the relative expression of microRNAs in pancreatic cancer compared to matched normal pancreas controls by real-time RT-PCR. Figure 2B shows the Northern blot of five fresh pancreatic cancer samples and two unmatched normal pancreas control for *miR-21.*

Figure 3 is a graph that shows a Kaplan-Meier overall survival curve for patients with pancreatic cancer based on relative expression of *miR-196a-2.*

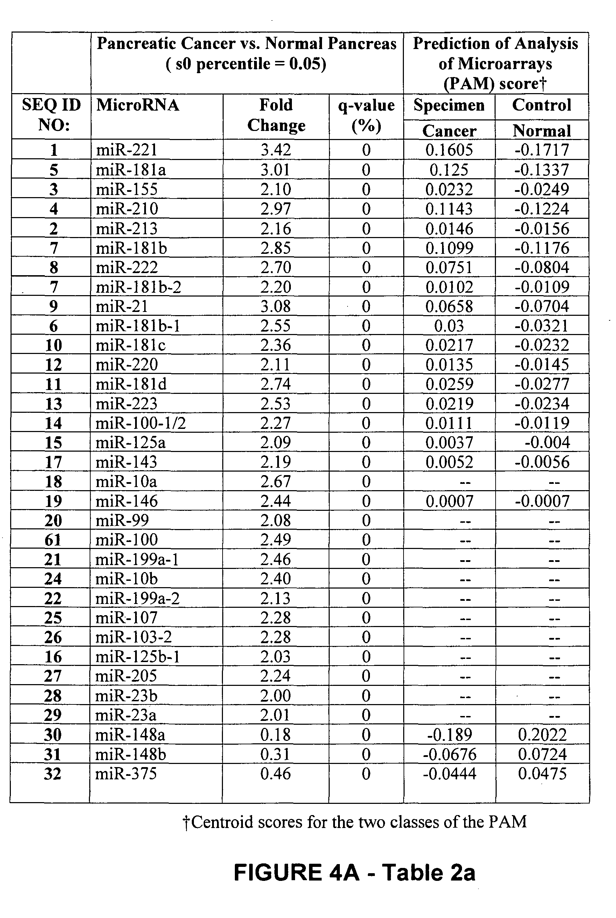

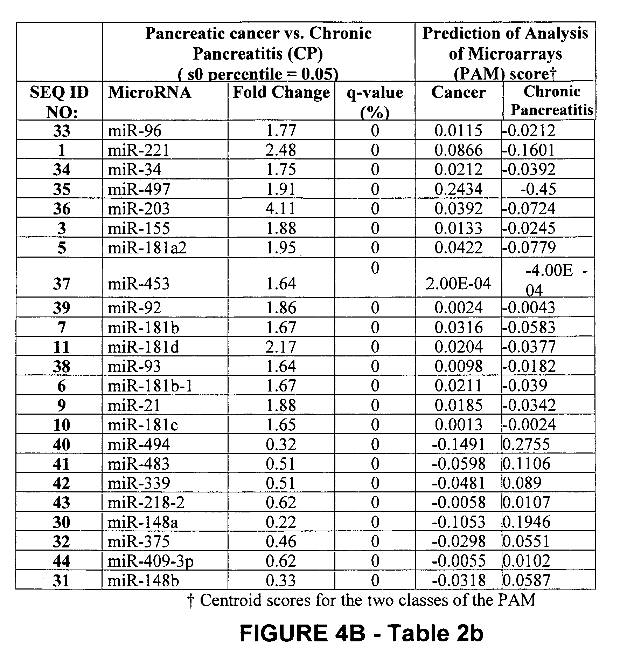

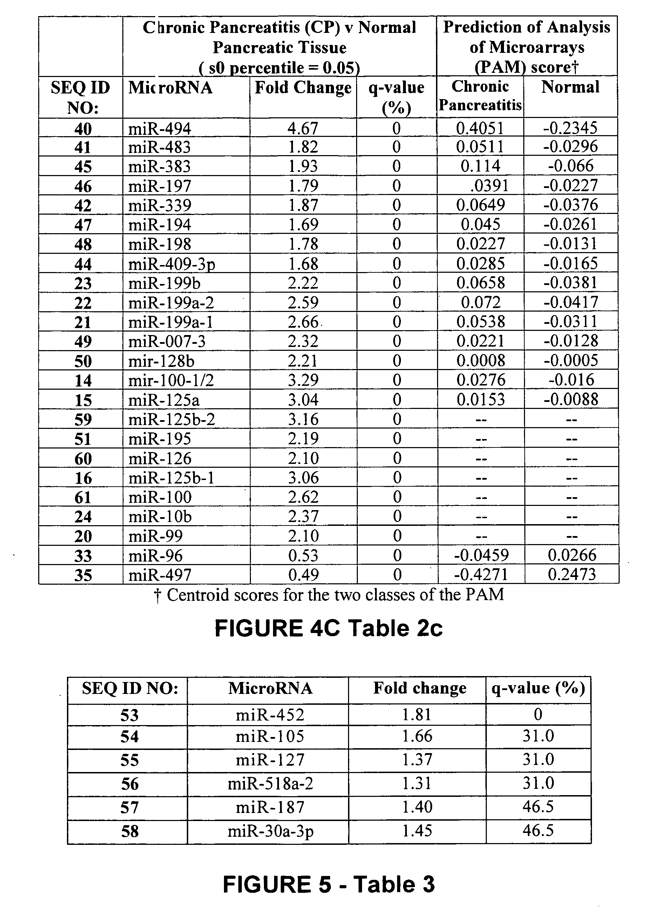

Figures 4A, 4B and 4C show Table 2a, 2b and 2c listing differentially expressed miRNAs and class predictors for pancreatic cancer (P), chronic pancreatitis (CP), and normal pancreatic function (NP) and their relative expression. Fold change is presented as actual change in expression.

Figure 5 shows Table 3 that includes differentially express mature microRNAs by SAM in node-positive patients with at least 24 months survival compared to those dying of disease within 24 months. Fold change is presented as actual change in expression. SAM variables were set at default (minimum nil fold change, 100 permutation, and s0 percentile of 0.05).

### DETAILED DESCRIPTION

The present invention will now be described with occasional reference to the specific embodiments of the invention. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to that this invention belongs. The terminology used in the description of the invention herein is for describing particular embodiments only and is not intended to be limiting of the invention. As used in the description of the invention and the appended claims, the singular forms "a," "an," and "the" are intended to include the plural forms as well, unless the context clearly indicates otherwise.

Unless otherwise indicated, all numbers expressing quantities of ingredients, properties such as molecular weight, reaction conditions, and so forth as used in the specification and claims are to be understood as being modified in all instances by the term "about." Accordingly, unless otherwise indicated, the numerical properties set forth in the following specification and claims are approximations that may vary depending on the desired properties sought to be obtained in embodiments described herein. Notwithstanding that the numerical ranges and parameters setting forth the broad scope of the invention are approximations, the numerical values set forth in the specific examples are reported as precisely as possible. Any numerical values, however, inherently contain certain errors necessarily resulting from error found in their respective measurements.

The present invention may be understood more readily by reference to the following detailed description and the Examples included herein. However, before the present methods, compounds and compositions are disclosed and described, it is to be understood that this invention is not limited to specific methods, specific cell types, specific host cells or specific conditions, etc., as such may, of course, vary, and the numerous modifications and variations therein will be apparent to those skilled in the art. It is also to be understood that the terminology used herein is for the purpose of describing specific embodiments only and is not intended to be limiting.

### Definitions

"Chemosensitivity" refers to the propensity of a cell to be affected by a cytotoxic agent, wherein a cell may range from sensitive to resistant to such an agent. The expression of a chemosensitivity gene, either alone or in combination with other factors or gene expression products, can be a marker for or indicator of chemosensitivity.

"Chemosensitivity gene" refers to a gene whose protein product influences the chemosensitivity of a cell to one or more cytotoxic agents. For example, along a scale that is a continuum, relatively high expression of a given gene in drug-sensitive cell lines is considered a positive correlation, and high expression in drug resistant cells is considered a negative correlation. Thus, negative correlation indicates that a chemosensitivity gene is associated with resistance of a cancer cell to a drug, whereas positive correlation indicates that a chemosensitivity gene is associated with sensitivity of a cancer cell to a drug. Chemosensitivity genes may themselves render cells more sensitive or more resistant to the effects of one or more cytotoxic agents, or may be associated with other factors that directly influence chemosensitivity. That is, some chemosensitivity genes may or may not directly participate in rendering a cell sensitive or resistant to a drug, but expression of such genes may be related to the expression of other factors which may influence chemosensitivity. Expression of a chemosensitivity gene can be correlated with the sensitivity of a cell or cell type to an agent, wherein a negative correlation may indicate that the gene affects cellular resistance to the drug, and a positive correlation may indicate that the gene affects cellular sensitivity to a drug.

It is to be noted herein that the specification lists the accession numbers for the known genes, whereby the full sequences of the genes may be referenced.

"Array" or "microarray" refers to an arrangement of hybridizable array elements, such as polynucleotides, which may be on a substrate. The arrangement of polynucleotides may be ordered. The array elements may be arranged so that there are at least ten or more different array elements, or at least 100 or more array elements. Furthermore, the hybridization signal from each of the array elements may be individually distinguishable. The array elements may comprise nucleic acid molecules. The array may comprise probes to two or more chemosensitivity genes, or may comprise probes to 10, 15, 20, 25, 30, 35, 40, 45, 50, 55, 60, 65, 70, 75, 80, 85, 90, 95, 100, 150, 200, 250 or more chemosensitivity genes. The array may comprise probes to genes that encode products other than chemosensitivity proteins. The array may comprise probes to 5, 10, 20, 30, 40, 50, 60, 70, 80, 90, 100, or more genes that encode products other than chemosensitivity proteins.

"Gene," when used herein, broadly refers to any region or segment of DNA associated with a biological molecule or function. Thus, genes include coding sequence, and may further include regulatory regions or segments required for their expression. Genes may also include non-expressed DNA segments that, for example, form recognition sequences for other proteins. Genes can be obtained from a variety of sources, including cloning from a source of interest or synthesizing from known or predicted sequence information, and may include sequences encoding desired parameters.

"Hybridization complex" refers to a complex between two nucleic acid molecules by virtue of the formation of hydrogen bonds between purines and pyrimidines.

"Identical" or percent "identity," when used herein in the context of two or more nucleic acid or polypeptide sequences, refer to two or more sequences or subsequences that may be the same or have a specified percentage of amino acid residues or nucleotides that are the same, when compared and aligned for maximum correspondence. For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

"Isolated," when used herein in the context of a nucleic acid or protein, denotes that the nucleic acid or protein is essentially free of other cellular components with that it is associated in the natural state. It is preferably in a homogeneous state although it can be in either a dry or aqueous solution. Purity and homogeneity are typically determined using analytical chemistry techniques such as polyacrylamide gel electrophoresis or high performance liquid chromatography. A protein that is the predominant molecular species present in a preparation is substantially purified. An isolated gene is separated from open reading frames that flank the gene and encode a protein other than the gene of interest.

"Marker," or "Biomarker" as used herein in reference to a chemosensitivity gene, means an indicator of chemosensitivity. A marker may either directly or indirectly influence the chemosensitivity of a cell to a cytotoxic agent, or it may be associated with other factors that influence chemosensitivity.

"Nucleic acid," when used herein, refers to deoxyribonucleotides or ribonucleotides, nucleotides, oligonucleotides, polynucleotide polymers and fragments thereof in either single- or double-stranded form. A nucleic acid may be of natural or synthetic origin, double-stranded or single-stranded, and separate from or combined with carbohydrate, lipids, protein, other nucleic acids, or other materials, and may perform a particular activity such as transformation or form a useful composition such as a peptide nucleic acid (PNA). Unless specifically limited, the term encompasses nucleic acids containing known analogues of natural nucleotides that have similar binding properties as the reference nucleic acid and may be metabolized in a manner similar to naturally-occurring nucleotides. Unless otherwise indicated, a particular nucleic acid sequence also implicitly encompasses conservatively modified variants thereof (e.g. degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (Batzer et al. (1991) Nucleic Acid Res. 19: 5081; Ohtsuka et al. (1985) J. Biol. Chem. 260: 2605-2608; Cassol et al. (1992); Rossolini et al. (1994) Mol. Cell. Probes 8: 91-98). The term nucleic acid is used interchangeably with gene, cDNA, and mRNA encoded by a gene.

An "Oligonucleotide" or "oligo" is a nucleic acid and is substantially equivalent to the terms amplimer, primer, oligomer, element, target, and probe, and may be either double or single stranded.

"Plurality" refers to a group of at least two or more members.

"Polynucleotide" refers to nucleic acid having a length from 25 to 3,500 nucleotides.

"Probe" or "Polynucleotide Probe" refers to a nucleic acid capable of hybridizing under stringent conditions with a target region of a target sequence to form a polynucleotide probe/target complex. Probes comprise polynucleotides that are 15 consecutive nucleotides in length. Probes may be 15, 16, 17, 18 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 5, 6, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 polynucleotides in length. Probes may be 70 nucleotides in length. Probes may be less than 100% complimentary to a target region, and may comprise sequence alterations in the form of one or more deletions, insertions, or substitutions, as compared to probes that are 100% complementary to a target region.

"Purified," when used herein in the context of nucleic acids or proteins, denotes that a nucleic acid or protein gives rise to essentially one band in an electrophoretic gel. Particularly, it means that the nucleic acid or protein is at least 50, 55, 60, 65, 70, 75, 80, 85, 90, 91, 92, 93, 94, 95, 96, 97, 98, 99 or 100% pure with respect to the presence of any other nucleic acid or protein species.

"Sample" refers to an isolated sample of material, such as material obtained from an organism, containing nucleic acid molecules. A sample may comprise a bodily fluid; a cell; an extract from a cell, chromosome, organelle, or membrane isolated from a cell; genomic DNA, RNA, or cDNA in solution or bound to a substrate; or a biological tissue or biopsy thereof. A sample may be obtained from any bodily fluid (blood, urine, saliva, phlegm, gastric juices, etc.), cultured cells, biopsies, or other tissue preparations.

"Stringent hybridization conditions" and "stringent hybridization wash conditions" in the context of nucleic acid hybridization experiments such as Southern and northern hybridizations are sequence dependent, and are different under different environmental parameters. Nucleic acids having longer sequences hybridize specifically at higher temperatures. An extensive guide to the hybridization of nucleic acids is found in Tijssen (1993) Laboratory Techniques in Biochemistry and Molecular Biology-Hybridization with Nucleic Acid Probes part I chapter 2 "Overview of principles of hybridization and the strategy of nucleic acid probe assays," Elsevier, N.Y. Generally, highly stringent hybridization and wash conditions are selected to be 5°C lower than the thermal melting point (Tₘ) for the specific sequence at a defined ionic strength and pH. Typically, under "stringent conditions" a probe will hybridize to its target subsequence, but to no other sequences. The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleic acids that have more than 100 complementary residues on a filter in a Southern or northern blot is 50% formamide with 1 mg of heparin at 42°C., with the hybridization being carried out overnight. An example of highly stringent wash conditions is 0.15 M NaCl at 72°C for 15 minutes. An example of stringent wash conditions is a 0.2.times.SSC wash at 65°C for 15 minutes (see, Sambrook, infra., for a description of SSC buffer). Often, a high stringency wash is preceded by a low stringency wash to remove background probe signal. An example medium stringency wash for a duplex of, e.g., more than 100 nucleotides, is 1.times.SSC at 45°C for 15 minutes. An example low stringency wash for a duplex of, e.g., more than 100 nucleotides, is 4-6xSSC at 40°C for 15 minutes. For short probes (e.g., 10 to 50 nucleotides), stringent conditions typically involve salt concentrations of less than 1.0 M Na ion, typically 0.01 to 1.0 M Na ion concentration (or other salts) at pH 7.0 to 8.3, and the temperature is typically at least 30°C. Stringent conditions can also be achieved with the addition of destabilizing agents such as formamide. In general, a signal to noise ratio of 2.times. (or higher) than that observed for an unrelated probe in the particular hybridization assay indicates detection of a specific hybridization. Nucleic acids that do not hybridize to each other under stringent conditions are still substantially similar if the polypeptides that they encode are substantially similar. This occurs, e.g., when a copy of a nucleic acid is created using the maximum codon degeneracy permitted by the genetic code.

"Substrate" refers to a support, such as a rigid or semi-rigid support, to which nucleic acid molecules or proteins are applied or bound, and includes membranes, filters, chips, slides, wafers, fibers, magnetic or nonmagnetic beads, gels, capillaries or other tubing, plates, polymers, and microparticles, and other types of supports, which may have a variety of surface forms including wells, trenches, pins, channels and pores.

"Target polynucleotide," as used herein, refers to a nucleic acid to which a polynucleotide probe can hybridize by base pairing and that comprises all or a fragment of a gene that encodes a protein that is a marker for chemosensitivity. In some instances, the sequences of target and probes may be 100% complementary (no mismatches) when aligned. In other instances, there may be up to a 10% mismatch. Target polynucleotides represent a subset of all of the polynucleotides in a sample that encode the expression products of all transcribed and expressed genes in the cell or tissue from which the polynucleotide sample is prepared. The gene products of target polynucleotides are markers for chemosensitivity; some may directly influence chemosensitivity by mediating drug transport. Alternatively, they may direct or influence cell characteristics that indirectly confer or influence sensitivity or resistance. For example, these proteins may function by establishing or maintaining the electrochemical gradient, or providing necessary nutrients for cells. Or they may be less directly involved and are expressed in conjunction with other factors that directly influence chemosensitivity.

"Target Region" means a stretch of consecutive nucleotides comprising all or a portion of a target sequence such as a gene or an oligonucleotide encoding a protein that is a marker for chemosensitivity. Target regions may be 15, 16, 17, 18 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 5, 6, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200 or more polynucleotides in length. Target regions may be 70 nucleotides in length, and lack secondary structure. Target regions may be identified using computer software programs such as OLIGO 4.06 software (National Biosciences, Plymouth Minn.), LASERGENE software (DNASTAR, Madison Wis.), MACDNASIS (Hitachi Software Engineering Co., San Francisco, Calif.) and the like.

DNA or RNA can be isolated from the sample according to any of a number of methods well known to those of skill in the art. For example, methods of purification of nucleic acids are described in Tijssen (1993) Laboratory Techniques in Biochemistry and Molecular Biology: Hybridization With Nucleic Acid Probes, Part I. Theory and Nucleic Acid Preparation, Elsevier, New York N.Y. In one case, total RNA is isolated using the TRIZOL reagent (Life Technologies, Gaithersburg Md.), and mRNA is isolated using oligo d(T) column chromatography or glass beads. Alternatively, when polynucleotide samples are derived from an mRNA, the polynucleotides can be a cDNA reverse transcribed from an mRNA, an RNA transcribed from that cDNA, a DNA amplified from that cDNA, an RNA transcribed from the amplified DNA, and the like. When the polynucleotide is derived from DNA, the polynucleotide can be DNA amplified from DNA or RNA reverse transcribed from DNA.

Suitable methods for measuring the relative amounts of the target polynucleotide transcripts in samples of polynucleotides are Northern blots, RT-PCR, or real-time PCR, or RNase protection assays. Fore ease in measuring the transcripts for target polynucleotides, any of a variety of arrays can be used.

The target polynucleotides may be labeled with one or more labeling moieties to allow for detection of hybridized probe/target polynucleotide complexes. The labeling moieties can include compositions that can be detected by spectroscopic, photochemical, biochemical, bioelectronic, immunochemical, electrical, optical or chemical means. The labeling moieties include radioisotopes, such as P.sup.32, P.sup.33 or S.sup.35, chemiluminescent compounds, labeled binding proteins, heavy metal atoms, spectroscopic markers, such as fluorescent markers and dyes, magnetic labels, linked enzymes, mass spectrometry tags, spin labels, electron transfer donors and acceptors, and the like

### Hybridization Complexes

Hybridization causes a denatured polynucleotide probe and a denatured complementary target polynucleotide to form a stable duplex through base pairing. Hybridization methods are well known to those skilled in the art (See, e.g., Ausubel (1997; Short Protocols in Molecular Biology, John Wiley & Sons, New York N.Y., units 2.8-2.11, 3.18-3.19 and 4-6-4.9). Conditions can be selected for hybridization where exactly complementary target and polynucleotide probe can hybridize, i.e., each base pair must interact with its complementary base pair. Alternatively, conditions can be selected where target and polynucleotide probes have mismatches but are still able to hybridize. Suitable conditions can be selected, for example, by varying the concentrations of salt in the prehybridization, hybridization and wash solutions, or by varying the hybridization and wash temperatures. With some membranes, the temperature can be decreased by adding formamide to the prehybridization and hybridization solutions.

Hybridization conditions are based on the melting temperature (Tₘ) the nucleic acid binding complex or probe, as described in Berger and Kimmel (1987) Guide to Molecular Cloning Techniques, Methods in Enzymology, vol 152, Academic Press. The term "stringent conditions, as used herein, is the "stringency" that occurs within a range from Tm-5 (5° below the melting temperature of the probe) to 20°C. below Tm. As used herein "highly stringent" conditions employ at least 0.2.times.SSC buffer and at least 65°C. As recognized in the art, stringency conditions can be attained by varying a number of factors such as the length and nature, i.e., DNA or RNA, of the probe; the length and nature of the target sequence, the concentration of the salts and other components, such as formamide, dextran sulfate, and polyethylene glycol, of the hybridization solution. All of these factors may be varied to generate conditions of stringency that are equivalent to the conditions listed above.

Hybridization can be performed at low stringency with buffers, such as 6xSSPE with 0.005% Triton X-100 at 37°C., which permits hybridization between target and polynucleotide probes that contain some mismatches to form target polynucleotide/probe complexes. Subsequent washes are performed at higher stringency with buffers, such as 0.5xSSPE with 0.005% Triton X-100 at 50°C, to retain hybridization of only those target/probe complexes that contain exactly complementary sequences. Alternatively, hybridization can be performed with buffers, such as 5xSSC/0.2% SDS at 60°C and washes are performed in 2.times.SSC/0.2% SDS and then in 0.1xSSC. Background signals can be reduced by the use of detergent, such as sodium dodecyl sulfate, Sarcosyl or Triton X-100, or a blocking agent, such as salmon sperm DNA.

### Array Construction

The nucleic acid sequences can be used in the construction of arrays, for example, microarrays. Methods for construction of microarrays, and the use of such microarrays, are known in the art, examples of which can be found in U.S. Pat. Nos. 5,445,934, 5,744,305, 5,700,637, and 5,945,334. Microarrays can be arrays of nucleic acid probes, arrays of peptide or oligopeptide probes, or arrays of chimeric probes--peptide nucleic acid (PNA) probes. Those of skill in the art will recognize the uses of the collected information.

One particular example, the in situ synthesized oligonucleotide Affymetrix GeneChip system, is widely used in many research applications with rigorous quality control standards. (Rouse R. and Hardiman G., "Microarray technology--an intellectual property retrospective," Pharmacogenomics 5:623-632 (2003).). Currently the Affymetrix GeneChip uses eleven 25-oligomer probe pair sets containing both a perfect match and a single nucleotide mismatch for each gene sequence to be identified on the array. Using a light-directed chemical synthesis process (photolithography technology), highly dense glass oligo probe array sets (>1,000,000 25-oligomer probes) can be constructed in a .about.3x3-cm plastic cartridge that serves as the hybridization chamber. The ribonucleic acid to be hybridized is isolated, amplified, fragmented, labeled with a fluorescent reporter group, and stained with fluorescent dye after incubation. Light is emitted from the fluorescent reporter group only when it is bound to the probe. The intensity of the light emitted from the perfect match oligoprobe, as compared to the single base pair mismatched oligoprobe, is detected in a scanner, which in turn is analyzed by bioinformatics software (http://www.affymetrix.com- ). The GeneChip system provides a standard platform for array fabrication and data analysis, which permits data comparisons among different experiments and laboratories.

Microarrays according can be used for a variety of purposes, as further described herein, including but not limited to, screening for the resistance or susceptibility of a patient to a drug based on the genetic expression profile of the patient.

### Methods of Predicting Response to Therapeutic Agents

In another aspect, there is provided herein a method of predicting the response of a patient, to treatment with a therapeutic agent. The method comprises contacting a polynucleotide sample obtained from the patient to polynucleotide probes to measure the levels of expression of one or a plurality of target polynucleotides. The expression levels of the target polynucleotides are then used to provide an expression profile for the patient that is then compared to the drug-gene correlations, wherein a positive correlation between a drug and a gene expressed in the patient indicates that the patient would be sensitive to the drug, and wherein a negative correlation between a drug and a gene expressed in the patient indicates that the patient would not be responsive to the drug.

### Methods of Identifying New Therapeutic Agents

Also provided herein are methods for identifying and characterizing new agents that modulate the ACE activity in a patient. The method comprises treating a sample of cells from a subject with an agent, and thereafter determining any change in expression of genes, such as the SNPs described herein, which are markers for chemosensitivity.

The method further comprises comparing the gene expression profiles of the control and treated cells to determine whether the agent alters the expression of any of the chemosensitive or chemoresistant genes. Separate cultures of cells may be exposed to different dosages of the candidate agent. The effectiveness of the agent's ability to alter chemosensitivity can be tested using standard assays. The agent is tested by conducting assays in that sample are co treated with the newly identified agent along with a previously known therapeutic agent. The choice of previously known therapeutic agent is determined based upon the gene-drug correlation between the gene or genes whose expression is affected by the new agent. Also provided are methods for identifying and characterizing new agents that modulate the chemosensitivity to ACE. The method comprises treating a sample of cells from the subject with an agent, which is capable of inhibiting the ACE activity implicated in chemosensitivity by correlation analysis between gene expression and drug potency.

Any cell line that is capable of being maintained in culture may be used in the method. The cell line may be a human cell line. According to one approach, RNA is extracted from such cells, converted to cDNA and applied to arrays to that probes have been applied, as described above.

### miRNA Expression Patterns in Pancreatic Adenocarcinoma

In one particular aspect, there is provided herein the identification of a global pattern of miRNA expression in pancreatic adenocarcinoma that accomplishes several goals. miRNAs are defined that can discriminate pancreatic cancer from normal pancreas. Since pancreatic cancer often occurs in a background of chronic pancreatitis, chronic pancreatitis specimens are utilized as a second control. A separate pattern of miRNA expression is used to delineate patients more likely to achieve long-term survival from those with shorter survival. The miRNA(s) whose expression would correlate with survival are identified.

As used herein interchangeably, a "miR gene product," "microRNA," "miR," or "miRNA" refers to the unprocessed or processed RNA transcript from a miR gene. As the miR gene products are not translated into protein, the term "miR gene products" does not include proteins. The unprocessed miR gene transcript is also called a "miR precursor," and typically comprises an RNA transcript of about 70-100 nucleotides in length. The miR precursor can be processed by digestion with an RNAse (for example, Dicer, Argonaut, RNAse III (e.g., *E. coli* RNAse III)) into an active 19-25 nucleotide RNA molecule. This active 19-25 nucleotide RNA molecule is also called the "processed" miR gene transcript or "mature" miRNA.

The active 19-25 nucleotide RNA molecule can be obtained from the miR precursor through natural processing routes (e.g., using intact cells or cell lysates) or by synthetic processing routes (e.g., using isolated processing enzymes, such as isolated Dicer, Argonaut, or RNAse III). It is understood that the active 19-25 nucleotide RNA molecule can also be produced directly by biological or chemical synthesis, without having to be processed from the miR precursor. When a micro RNA is referred to herein by name, the name corresponds to both the precursor and mature forms, unless otherwise indicated.

There is provided herein methods of diagnosing whether a subject has, or is at risk for developing, pancreatic cancer, comprising measuring the level of at least one miR gene product in a test sample from the subject and comparing the level of the miR gene product in the test sample to the level of a corresponding miR gene product in a control sample. As used herein, a "subject" can be any mammal that has, or is suspected of having, pancreatic cancer. The subject may be a human who has, or is suspected of having, pancreatic cancer.

The pancreatic cancer can be any form of pancreatic cancer, for example, pancreatic cancers of differing histology (e.g., exocrine tumors, endocrine tumors, carcinomas, lymphomas). The pancreatic cancer that is diagnosed may be a pancreatic adenocarcinoma). The pancreatic cancer that is diagnosed may be selected from the group consisting of a pancreatic endocrine tumor (PET) and a pancreatic exocrine tumor (e.g., an adenocarcinoma). Furthermore, as described herein, the pancreatic cancer may be associated with a particular prognosis (e.g., low survival rate, fast progression).

### EXAMPLES

In order that the invention disclosed herein may be more efficiently understood, examples are provided below. It should be understood that these examples are for illustrative purposes only and are not to be construed as limiting the invention in any manner. Throughout these examples, molecular cloning reactions, and other standard recombinant DNA techniques, were carried out according to methods described in Maniatis et al., Molecular Cloning--A Laboratory Manual, 2nd ed., Cold Spring Harbor Press (1989), using commercially available reagents, except where otherwise noted.

Methods and compositions for altering genome amounts in a target cell are provided. In the subject methods, the activity of a miRNA is altered in a manner sufficient to alter the amount of genome in the target cell; for example, by introducing a miRNA regulatory agent in the target cell. Also provided are pharmaceutical compositions, kits and systems for use in practicing the subject methods. The subject invention finds use in a variety of applications, including the treatment of subjects suffering from a pancreatic disease condition

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Methods recited herein may be carried out in any order of the recited events which is logically possible, as well as the recited order of events.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

As summarized above, this disclosure provides methods and compositions altering the amount of a target genome in a target cell. The subject methods are described first in greater detail, followed by a review of various representative applications in which this disclosure finds use as well as kits that find use in practicing the subject invention.

### miRNAs in Ductal Adenocarcinoma

While global microRNA (miRNA) expression patterns of many embryologic, physiologic, and oncogenic processes have been described, description of the role of miRNAs in ductal adenocarcinoma of the pancreas is lacking. Thus, in one aspect, there is provided a definition of the expression pattern of miRNAs in pancreatic cancer and a compare it to those of normal pancreas and chronic pancreatitis.

Broadly, RNA harvested from resected pancreatic cancers and matched normal adjacent pancreas as well as chronic pancreatitis specimens was hybridized to miRNA microarrays. Significance of Analysis of Microarrays (SAM) and Prediction of Analysis of Microarrays (PAM) were undertaken to identify miRNAs predictive of tissue type and prognosis, p-values were calculated by using t test adjusted for multiple testing. Kaplan-Meier survival curves were constructed thresholded on mean miRNA expression (high vs. low) and compared by log-rank analysis.

The patient population was comprised of consecutive patients with ductal adenocarcinoma of the pancreas (N=65) or chronic pancreatitis (N=42). The intervention(s) included curative radical pancreatectomy was undertaken in all patients. Patients with pancreatic cancer were chemotherapy naive.

Identified were differentially expressed miRNAs that can discriminate pancreatic cancer from normal pancreatic function and/or chronic pancreatitis as well as identifying a pattern of miRNA expression that was predictive of long-term (i.e. >24 months) survival.

Identified were 21 miRNAs with increased expression and four with decreased expression that correctly classified pancreatic cancer from normal pancreas in 90% of samples by cross validation. Fifteen overexpressed and nine underexpressed miRNAs discriminated pancreatic cancer from chronic pancreatitis with 93% accuracy. A subgroup of six miRNAs was able to differentiate long-term (i.e. > 24 months) survivors with node-positive disease from those dying within 24 months. Finally, high *miR-196a-2* expression was found to be predictive of poor survival (median 14.3 months [95% CI 12.4 to 16.2] vs. 26.5 [95% CI 23.4 to 29.6], p=0.009).

Pancreatic cancer may have a distinct miRNA expression pattern that may differentiate it from normal pancreatic function and chronic pancreatitis. The distinct miRNA expression pattern is useful to distinguish between long-term and short-term survivors.

### Material and Methods

### Tissue samples

After being granted exempt status by the institutional review board at the Ohio State University, specimens from 65 consecutive patients who underwent resection for ductal adenocarcinoma of the pancreas and 42 with chronic pancreatitis from January 2000 through December 2005 were identified from the archival files of the Ohio State University Department of Pathology. All cases were reviewed by one pathologist and the diagnoses confirmed. Three two millimeter cores were obtained from the microdissected paraffin blocks for pancreatic cancer and matched benign adjacent pancreas or for chronic pancreatitis. Benign adjacent pancreas was available from all pancreatic cancer specimens.

### MicroRNA microarray

Tissue cores were deparaffinized with xylene at 50°C for 3 minutes. Total RNA extraction was undertaken using the RecoverAll kit (Ambion, Inc., Austin, TX) according to manufacturer's instructions. RNA labeling and hybridization on miRNA microarray chips were done as previously described.⁷ Briefly, 5µg of total RNA from each sample was reverse transcribed by using biotin end-labeled random octamer oligonucleotide primer. Hybridization of biotin-labeled cDNA was carried out on a custom miRNA microarray chip (OSU_CCC version 3.0), which contain~1100 miRNA probes, including 326 human and 249 mouse miRNA genes, spotted in duplicates. The hybridized chips were washed and processed to detect biotin-containing transcripts by streptavidin-Alexa647 conjugate and scanned on an Axon 4000B (Axon Instruments, Sunnyvale, CA).

### Statistical and bioinformatics analysis

Microarray images were analyzed by using GENEPIX PRO 6.0 (Axon Instruments). Average values of the replicate spots of each miRNA were background subtracted, normalized, and subjected to further analysis. Normalization was performed by using per chip median normalization method and the median array.⁸ Finally, we selected the miRNAs measured as present in at least as many samples as the smallest class in the data set (25%). Absent calls were thresholded to 4.5 (log 2 scale) before statistical analysis, representing the average minimum intensity level detectable in the system. Greater than 95% of blank probes (i.e. negative controls) fall below the threshold value of 4.5. Differentially expressed miRNA between pancreatic cancer and normal pancreas, pancreatic cancer and chronic pancreatitis, and chronic pancreatitis and normal pancreas were identified by using the Significance Analysis of Microarrays (SAM) 3.0 application with a threshold difference in expression set to 2, s0 percentile set to 0.05 (default) and the number of permutations set to 100 (default).⁹ SAM calculates a score for each gene on the basis of the change of expression relative to the standard deviation of all measurements. Only mature miRNAs differentially expressed are reported. MiRNA signatures were determined by Prediction Analysis of Microarrays (PAM) which implements nearest shrunken centroids.¹⁰ The prediction error was calculated by means of 10-fold cross validation. For hierarchical analysis, we employed average linkage clustering of the microRNAs identified by SAM and PAM between normal pancreas and pancreatic cancer (Cluster 3.0). Java Treeview 1.0 was used for tree visualization.

To perform survival analysis and generate Kaplan-Meier survival curves, miRNA levels measured on the miRNA chips were converted into discrete variables by splitting the samples in two classes (high and low expression), using the respective mean level of miRNA expression as threshold. Survival curves were compared by log-rank analysis. Significance was accepted with 95% confidence.

### Quantitative RT-PCR

The single tube TaqMan miRNA Assay was used to detect and quantify mature miRNAs on Applied Biosystems Real-Time PCR instruments in accordance with manufacturer's instructions (Applied Biosystems, Foster City, CA). Normalization was performed with the small nuclear RNA U6 (RNU6B; Applied Biosystems). All RT reactions, including no-template controls and RT minus controls, were run in a GeneAmp PCR 9700 Thermocycler (Applied Biosystems). Gene expression levels were quantified using the ABI Prism 7900HT Sequence detection system (Applied Biosystems). Comparative real-time PCR was performed in triplicate, including no-template controls. Relative expression was calculated using the comparative Cₜ method.

Similarly, TaqMan Gene Expression Assay was undertaken using primers and probes (pre-designed, pre-optimized) for *KRAS* expression determination obtained from Applied Biosystems. One hundred fifty nanogram RNA was used per sample for the assays and 18S was used to normalize all RNA samples.

### Tissue Microarray (TMA)

Our method for TMA creation has been described.¹¹ Briefly, two tissue cores (2 mm diameter each) were punched out of each paraffin block used to obtain RNA for microRNA analysis and transferred to each of the recipient TMA blocks using a precision instrument (Beecher Instruments, Silver Spring, MD). Paraffin embedded tissue was cut at 4 microns and placed on positively charged slides then heated to 40°C for 30 minutes. After leveling paraffin and cores, the array was cooled to 4°C for 15 minutes.

### Immunohistochemistry (IHC)

The methods for immunohistochemistry have been described.¹¹ Primary antibodies for TP53 (catalog #M7001, clone DO-7, Dako, Carpinteria, CA), CDKN2 (catalog #CMC802, clone JC2, Cell Marque Corp., Rocklin, CA), and SMAD4/DPC4 (catalog #sc-7966, clone B-8, Santa Cruz Biotechnology, Inc., Santa Cruz, CA) were used at dilutions of 1:50, 1:20, and 1:100, respectively. Slides were counterstained in Richard Allen hematoxylin, dehydrated through graded ethanol solutions and cover-slipped. The positive and negative controls stained appropriately.

Staining for TP53 was considered positive if nuclear staining in at least 5% of cells was seen. Nuclear and cytoplasmic staining in at least 5% of cells was considered positive for CDKN2 and less than 10% of nuclear and cytoplasmic staining of cell for SMAD4/DPC4 was considered as loss of expression. All stains were read by a single pathologist (WLF) blinded to tumor stage and clinical characteristics.

### Results

Utilizing miRNA microarray⁸, differentially expressed miRNAs were identified between pancreatic cancers and matched adjacent normal pancreas, between pancreatic cancer and chronic pancreatitis, and between chronic pancreatitis and normal pancreas. SAM identified 31 miRNAs that were up-regulated in pancreatic cancers and three that were down-regulated compared to normal pancreas. When pancreatic cancer samples were compared to chronic pancreatitis, three miRNAs were overexpressed and four were underexpressed in cancers. Finally, 16 miRNAs showed increased expression in chronic pancreatitis compared to one that was decreased compared to normal pancreas.

Tables 2a, 2b, and 2c (shown in Figures 4A, 4B and 4C, respectively) lists differentially expressed miRNAs with at least a two-fold change in expression by SAM and additional miRNAs identified as class predictors by PAM (see below). One-third of miRNAs found to discriminate pancreatic cancer from normal pancreas also differentiated cancers from chronic pancreatitis (Figures 1A-C, also showing Tables 1a, 1b, 1c). No miRNAs were common between all three groups of samples.

Figures 1A, 1B and 1C show a Venn diagram illustrating the relationships between sets of miRNAs found to be differentially expressed by pairwise comparisons between tissue types; and show Tables 1a, 1b and 1c listing the sets of miRNAs found to be differentially expressed by pairwise comparisons between tissue types.

Circles include the total number of differentially expressed miRNAs in the pairwise comparison indicated. Intersecting areas demonstrate the number of differentially expressed miRNAs in common between each comparison. The common miRNAs are listed for each set. NP, normal pancreas; P, pancreatic cancer; CP, chronic pancreatitis.

Cluster analysis based upon miRNAs differentially expressed between chronic pancreatitis, normal pancreas, and pancreatic cancer demonstrated a general distinction between each sample type (data not shown). The expression patterns appeared to be most similar between chronic pancreatitis and normal pancreas with a more clear distinction between these benign tissues and pancreatic cancer. The majority of pancreatic cancers clustered together with some exceptions including a group of eight cancers clustering among the normal pancreas and chronic pancreatitis samples. This latter group had similar clinicopathologic features as the remainder of the cancers with survival which was 50% longer but not statistically significant (median 23.1 months [95%CI 19.6 to 26.6] vs. 15.2 [95%CI 10.9 to 19.5], p=0.15). This group of eight tumors had significantly lower levels of miR-21 expression compared to the other pancreatic cancers (median 10.9 vs. 8.3. p=0.0003).

PAM allowed classification of each sample by tissue type based upon miRNA expression levels (Figures 4A, 4B, 4C, showing Tables 2a, 2b, 2c). A subset of 21 overexpressed and four underexpressed miRNAs were identified that could correctly discriminate pancreatic cancer from normal pancreas by cross validation testing in 90% of samples. When comparison was made between chronic pancreatitis and pancreatic cancer, samples were correctly classified in 93% based upon 15 overexpressed and nine underexpressed miRNAs in cancer.

Comparison between chronic pancreatitis and normal pancreas identified 15 5 miRNAs with increased expression and two with decreased expression. This pattern of expression correctly discriminated chronic pancreatitis from normal pancreas in all samples. Finally, 95% of pancreatic cancer samples were classified correctly when compared to both chronic pancreatitis and normal pancreas together.

To confirm the microarray findings, quantitative real-time PCR was undertaken in eight pancreatic cancer samples and eight matched normal pancreas controls for *miR-21, miR-155, miR-221, miR-222, miR-181a, miR-181b,* and *miR-181d.*

All of these miRNAs were overexpressed in tumor samples relative to normal pancreas (Figure 2A).

Northern blot analysis for *miR-21* in five additional fresh pancreatic cancer samples also confirmed increased expression compared to two unmatched fresh normal pancreas controls (Figure 2B).

To determine the impact of miRNA expression on survival we analyzed our microarray data using two methods. First, we wanted to determine if the absolute level of miRNA expression could discriminate between short-term and long-term survivors with node-positive disease. Given that patients with pancreatic cancer metastatic to regional lymph nodes still alive two years after resection are often considered as long-term survivors, we compared microarray data for node-positive patients with greater than 24 month's survival to those dying of disease within 24 months. SAM identified six miRNAs that were differentially overexpressed in the patients with longer survival, as shown in Table 3 in Figure 5.

Next, we wanted to determine the survival based upon the relative expression of miRNAs. Kaplan-Meier survival curves were generated and compared by Log-Rank analysis using the binomial variable of high or low expression relative to the mean expression of each miRNA on the microarray. Based upon this, two miRNAs of interest were identified: *miR-196a-2* **[SEQ ID NO: 62]** and *miR-219* **[SEQ ID NO: 64]**.

Tumors with high *miR-196a-2* expression had a median survival of 14.3 months (95%CI 12.4 to 16.2) compared to 26.5 months (95%CI 23.4 to 29.6) for those with low expression (p=0.009). High *miR-196a-2* expression, which was seen in 75% of tumors, resulted in two-year survival of 17% compared to 64% for low expression (Figure 3).

Median survival in patients with high *miR-219* expression was 13.6 months (95%CI 11.8 to 15.4) compared to 23.8 months (95%CI 18.7 to 28.9) for those with low expression with two-year survivals of 25% and 49%, respectively (p=0.067). Median survival for all patients was 15.5 months (95%CI 9.9 to 21.1) with two- and five-year survivals of 33% and 12.5%, respectively. Of note, nodal status, T stage, and histologic grade were not predictive of survival (data not shown).

We next sought to correlate the expression of common genetic abnormalities seen in pancreatic cancer with survival and microRNA expression. Increased TP53 expression was seen in 31 of 54 (57%) tumors. Loss of CDKN2 expression was seen in 49 of 56 (88%) of tumors while SMAD4/DPC4 expression was lost in 39 of 56 (70%). No correlation was found with survival or any of the microRNAs listed in Table 3 (shown in Figure 5) including miR-196a-2. In addition, KRAS mutation was identified in eight often (80%) of tumors evaluated by gene expression analysis but did not correlate with microRNA expression.

### Discussion

Aberrant miRNA expression patterns have been described in a variety of hematologic and solid organ malignancies. Identified is a global expression pattern of miRNAs which can differentiate ductal adenocarcinomas of the pancreas from normal pancreas and chronic pancreatitis with 95% accuracy. As well we have identified a miRNA, *miR-196a-2,* which may significantly impact survival.

For this series of experiments two controls were chosen for comparison: adjacent normal pancreas and chronic pancreatitis. Although our tumor samples were microdissected, contamination with surrounding inflammatory changes common in pancreatic cancers is inevitable. Still, only seven miRNAs (*miR-99, miR-100, miR-100-1*/*2, miR-125a, miR-125b-1, miR-199a-1, miR-199a-2*) which were found to be overexpressed in cancers compared to normal pancreas were also overexpressed in chronic pancreatitis.

While the overexpression of these miRNAs in cancers and chronic pancreatitis may suggest a common inciting event for neoplastic growth, the possibility of contamination cannot be excluded. In general, normal pancreatic function and chronic pancreatitis tended to cluster together while remaining largely separate from the pancreatic cancers with few exceptions. Interestingly, one group of eight cancers did cluster with the benign pancreas samples. While this group of patients did not have a significant improvement in survival compared to the others, their nearly two-year median survival is longer than most reports for pancreatic cancer.

Noteworthy within this smaller group is the cluster of *miR-23, miR-103,* and *miR-107* which was lower in these eight cancers than seen in the remaining cancers or the majority of benign pancreas (data not shown). These miRNAs, among others, have recently been shown to be induced by hypoxia in cancer cells via a hypoxia-inducible factor (HIF)-dependent mechanism.¹² In fact, the majority of the described hypoxia-related miRNAs are differentially overexpressed in our pancreatic cancers. Given the association between HIF and pancreatic cancer aggressiveness, decreased expression of these hypoxia related miRNAs may prove important for survival in a subset of patients.

Several miRNAs commonly associated with malignancy were identified in our pancreatic cancers as significantly deregulated. Most notably, *miR-21* and *miR-155* were uniquely overexpressed in pancreatic cancer versus normal pancreas and chronic pancreatitis. *MiR-21* has been suggested to play an important role in preventing apoptosis, thus functioning as a protooncogene¹³ and has been shown to be overexpressed in cancers of the lung, stomach, breast, colon, and prostate as well as being expressed in pancreatic neuroendocrine tumors.^{4, 7} While the role of *miR-21* in neoplasia has not been fully elucidated, its inhibition using miRNA-specific antisense oligonucleotides increases *in vitro* susceptibility of cholangiocarcinoma cells to gemcitabine. ¹⁴ *MiR-155* is also overexpressed in solid tumors such as colon, lung, and breast cancers^{4, 7} while being associated with the activated B-cell type of diffuse large B-cell lymphoma as well as Hodgkin's and Burkitt's lymphoma.^{15, 16} It has also been shown to be involved in leukemogenesis in transgenic mice.¹⁷

The most consistently highly expressed miRNA in our pancreatic cancers was *miR-221* when compared to normal pancreas and chronic pancreatitis. While this association has not been shown in gastrointestinal tumors previously, *miR-221* expression is important in thyroid cancer and is suggested to play a role in angiogenesis.^{18, 19} Working together with *miR-222,* which was also found to be overexpressed in pancreatic cancers we studied, *miR-221* targets the receptor for Stem Cell Factor, KIT.

Far fewer miRNAs were down-regulated in pancreatic cancer. Notable of these was *miR-375* which is found in abundance in pancreatic islets but not in the exocrine pancreas.²⁰ It stands to reason that this miRNA would be significantly underexpressed in our pancreatic cancers as they were all derived from the exocrine pancreas resulting in obliteration of the intervening islets.

The miRNA expression patterns of 40 pancreatic endocrine tumors (PETs) and four acinar carcinomas are compared to normal pancreas.²¹ In that study, 87 miRNAs were differentially overexpressed in tumors and eight were underexpressed relative to normal pancreas. This is markedly more than the 31 overexpressed miRNAs and three underexpressed miRNAs identified in the pancreatic adenocarcinomas of ductal origin. Given the difference in derivation of PETs compared to ductal adenocarcinomas, this wide variety in miRNA expression is not unexpected. Similar findings have been reported utilizing Affymetrix gene arrays.²² Similar to our findings in ductal adenocarcinoma, *miR-*21 appears to be important in PETs. In the endocrine tumors, however, *miR-21* correlated with more aggressive tumors as signified by an increased proliferation index by Ki67 and the presence of liver metastases. Similarly, *miR-21* expression was significantly lower in the eight cancers reported herein that clustered with the benign pancreas specimens suggesting its role in tumor aggression. *MiR-155,* on the other hand, was underexpressed in PETs relative to normal pancreas whereas we found it to be overexpressed in ductal adenocarcinomas. This discrepancy, again, emphasizes the differences in cell origin between the two tumor types.

Given the dismal prognosis typically associated with pancreatic cancer, we sought to identify a miRNA expression profile that could discriminate between high-risk patients who could be considered long-term (i.e. greater than 24 months) and short-term survivors. A group of six miRNAs were identified (Figure 5, Table 3).

*MiR-127* is interesting since it is located within a CpG island on chromosome 14 and shown to be silenced in cancers of the prostate and colon.²³ In our pancreatic cancers, *miR-127* expression was increased in nearly half of the tumors while it was decreased in the other half. While, *miR-127* expression alone does not significantly impact survival but, when taken with the other miRNAs listed in Figure 5-Table 3, it can be used predict long-term survivors.

Only one miRNA was identified that significantly predicted duration of survival, *miR-196a-2.* This miRNA was not identified by SAM to discriminate between the qualitative distinction of "long-" and "short-term" survivors. While a direct association with malignancy has not been described for *miR-196,* it does appear to perfectly interact with, and degrade *HOXB8,* a member of the homeobox family cluster involved in various crucial development programs in animals²⁴, including the endocrine pancreas.²⁵ In our patients, 75% of tumors expressed *miR-196a-2* at a level above the mean for the group. Although this miRNA did not help differentiate pancreatic cancers from normal pancreas or chronic pancreatitis, it can be a useful predictor for survival.

The samples used are representative of typical ductal adenocarcinomas, as demonstrated by assaying for the four most common genetic abnormalities seen in pancreatic cancer: *TP53, CDKN2, SMAD4,* and *KRAS.* Alterations in these genes of interest seen in our tumors were similar to those described in the literature.²⁶⁻²⁸ Similar to earlier reports, these tumor suppressor and oncogenes did not correlate with survival in our patients, nor did they correlate with miRNA expression.

These results are believed to now show global expression patterns of miRNAs in pancreatic adenocarcinoma. Such patterns can be utilized to direct therapy in patients with metastatic tumors of unknown primary or to help discriminate between benign and malignant neoplasms that would otherwise be indeterminate by routine histologic and immunohistochemical analysis. This data such can also be useful to differentiate between patients with better or worse prognoses in order to help guide the clinician when determining who should or should not receive aggressive therapy. Aside from these diagnostic and prognostic examples of how microRNA expression patterns can be utilized clinically, the ability of microRNAs to effect multiple genes in various pathways make them useful for anti-tumoral therapies.

### Applications

The above-described methods find use in a variety of different applications, representative types of which are described herein

### Utility

The subject methods find use in the treatment of a variety of different conditions in which the reduction of a target genome amount in a target cell or host comprising the same is desired. The subject methods find use in the treatment of a host suffering from pancreatic cancer mediated disease condition. By treatment is meant that at least an amelioration of the symptoms associated with the condition afflicting the host is achieved, where amelioration is used in a broad sense to refer to at least a reduction in the magnitude of a parameter, e.g. symptom, associated with the condition being treated. As such, treatment also includes situations where the pathological condition, or at least symptoms associated therewith, are completely inhibited, e.g. prevented from happening, or stopped, e.g. terminated, such that the host no longer suffers from the condition, or at least the symptoms that characterize the condition.

A variety of hosts are treatable according to the subject methods. Generally such hosts are "mammals" or "mammalian," where these terms are used broadly to describe organisms which are within the class mammalia, including the orders. carnivore (e.g., dogs and cats), rodentia (e.g., mice, guinea pigs, and rats), and primates (e.g., humans, chimpanzees, and monkeys). The hosts may be humans.

The present disclosure identifies the global changes in gene expression associated with pancreatic cancer by examining gene expression in tissue from normal pancreas, metastatic malignant pancreatic cancer and chronic pancreatitis.

The present disclosure also identifies expression profiles which serve as useful diagnostic markers as well as markers that can be used to monitor disease states, disease progression, drug toxicity, drug efficacy and drug metabolism.

The disclosure includes methods of diagnosing the presence or absence of pancreatic cancer in a patient comprising the step of detecting the level of expression in a tissue sample of two or more genes from Tables 1-2 wherein differential expression of the genes in Tables 1-2 is indicative of pancreatic cancer. One or more genes may be selected from a group consisting of the genes listed in Table 2.

The disclosure also includes methods of detecting the progression of pancreatic cancer and/or differentiating cancerous disease from chronic inflammation. For instance, methods disclosed herein include detecting the progression of pancreatic cancer in a patient comprising the step of detecting the level of expression in a tissue sample of two or more genes from Tables 1-2; wherein differential expression of the genes in Tables 1-2 is indicative of pancreatic cancer progression. One or more genes may be selected from a group consisting of the genes listed in Table 2.

The present disclosure provides a method of monitoring the treatment of a patient with pancreatic cancer, comprising administering a pharmaceutical composition to the patient, preparing a gene expression profile from a cell or tissue sample from the patient and comparing the patient gene expression profile to a gene expression from a cell population comprising normal pancreatic cells or to a gene expression profile from a cell population comprising pancreatic cancer cells or to both. The gene profile may include the expression level of one or more genes in Tables 1-3. One or more genes may be selected from a group consisting of the genes listed in Table 3.

The present disclosure provides a method of treating a patient with pancreatic cancer, comprising administering to the patient a pharmaceutical composition, wherein the composition alters the expression of at least one gene in Tables 1-3, preparing a gene expression profile from a cell or tissue sample from the patient comprising tumor cells and comparing the patient expression profile to a gene expression profile from an untreated cell population comprising pancreatic cancer cells. One or more genes may be selected from a group consisting of the genes listed in Table 3.

The present disclosure provides a method of diagnosing pancreatic cancer in a patient, comprising detecting the level of expression in a tissue sample of two or more genes from Tables 1-3, wherein differential expression of the genes in Tables1-3 is indicative of pancreatic cancer. One or more genes may be selected from a group consisting of the genes listed in Table 3.

The present disclosure provides a method of detecting the progression of pancreatic cancer in a patient, comprising detecting the level of expression in a tissue sample of two or more genes from Tables1-3; wherein differential expression of the genes in Table 1-3 is indicative of pancreatic cancer progression. One or more genes may be selected from a group consisting of the genes listed in Table 3.

The present disclosure provides a method of monitoring the treatment of a patient with a metastatic pancreatic tumor, comprising administering a pharmaceutical composition to the patient, preparing a gene expression profile from a cell or tissue sample from the patient and comparing the patient gene expression profile to a gene expression from a cell population comprising normal pancreatic cells or to a gene expression profile from a cell population comprising metastatic pancreatic tumor cells or to both. The method may include detecting the expression level of one or more genes selected from the genes listed in Tables 1-3. One or more genes may be selected from a group consisting of the genes listed in Table 3.

Also provided is a method of treating a patient with a metastatic pancreatic tumor, comprising administering to the patient a pharmaceutical composition, wherein the composition alters the expression of at least one gene in Tables 1-3, preparing a gene expression profile from a cell or tissue sample from the patient comprising metastatic pancreatic tumor cells and comparing the patient expression profile to a gene expression profile from an untreated cell population comprising metastatic pancreatic tumor cells. One or more genes may be selected from a group consisting of the genes listed in Table 3.

The present disclosure also includes methods of differentiating pancreatic cancer from other pancreatic disorders in a patient comprising the step of detecting the level of expression in a tissue sample of two or more genes from Tables 1-3; wherein differential expression of the genes in Tables 1-3 is indicative of pancreatic cancer rather than another pancreatic disorder.

The present disclosure further includes methods of screening for an agent capable of modulating the onset or progression of pancreatic cancer, comprising the steps of exposing a cell to the agent; and detecting the expression level of two or more genes from Table1-3. One or more genes may be selected from a group consisting of the genes listed in Table 3.

Any of the methods described above may include the detection of at least 2 genes from the tables. The methods may detect all or nearly all of the genes in the tables. One or more genes may be selected from a group consisting of the genes listed in Tables 1-3.

The present disclosure further includes compositions comprising at least two oligonucleotides, wherein each of the oligonucleotides comprises a sequence that specifically hybridizes to a gene in Tables 1-3 as well as solid supports comprising at least two probes, wherein each of the probes comprises a sequence that specifically hybridizes to a gene in Tables 1-3. One or more genes may be selected from a group consisting of the genes listed in Table 3.

The present disclosure further includes computer systems comprising a database containing information identifying the expression level in pancreatic tissue of a set of genes comprising at least two genes in Tables 1-3; and a user interface to view the information. One or more genes may be selected from a group consisting of the genes listed in Table 3. The database may further include sequence information for the genes, information identifying the expression level for the set of genes in normal pancreatic tissue and malignant tissue (metastatic and nonmetastatic) and may contain links to external databases such as GenBank; the databases maintained by the National Center for Biotechnology Information or NCBI (ncbi.nlm.nih.gov/Entrez/). Other external databases that may be used include those provided by Chemical Abstracts Service (stnweb.cas.org/) and Incyte Genomics (incyte.com/sequence/index.shtml).

The present disclosure further comprises kits useful for the practice of one or more of the methods disclosed herein. A kit may contain one or more solid supports having attached thereto one or more oligonucleotides. The solid support may be a high-density oligonucleotide array. Kits may further comprise one or more reagents for use with the arrays, one or more signal detection and/or array-processing instruments, one or more gene expression databases and one or more analysis and database management software packages.

The present disclosure further includes methods of using the databases, such as methods of using the disclosed computer systems to present information identifying the expression level in a tissue or cell of at least one gene in Tables 1-3, comprising the step of comparing the expression level of at least one gene in Tables 1-3 in the tissue or cell to the level of expression of the gene in the database. One or more genes may be selected from a group consisting of the genes listed in Table 3.

The present disclosure provides compositions and methods to detect the level of expression of genes that may be differentially expressed dependent upon the state of the cell, i.e., normal versus cancerous. As used herein, the phrase "detecting the level expression" includes methods that quantitate expression levels as well as methods that determine whether a gene of interest is expressed at all. Thus, an assay which provides a yes or no result without necessarily providing quantification of an amount of expression is an assay that requires "detecting the level of expression" as that phrase is used herein.

### Pharmaceutical Compositions

Also disclosed are pharmaceutical compositions containing the miRNA compounds employed in the subject methods. Accordingly, the compounds, e.g., in the form of a pharmaceutically acceptable salt, can be formulated for oral or parenteral administration for use in the subject methods, as described above.

By way of illustration, the compounds can be admixed with conventional pharmaceutical carriers and excipients (i.e., vehicles) and used in the form of aqueous solutions, tablets, capsules, elixirs, suspensions, syrups, wafers, and the like. Such pharmaceutical compositions contain from about 0.1 to about 90% by weight of the active compound, and more generally from about 1 to about 30% by weight of the active compound. The pharmaceutical compositions may contain common carriers and excipients. Non-limiting examples include corn starch or gelatin, lactose, dextrose, sucrose, microcrystalline cellulose, kaolin, mannitol, dicalcium phosphate, sodium chloride, and alginic acid. Disintegrators commonly used in the formulations include croscarmellose, microcrystalline cellulose, corn starch, sodium starch glycolate and alginic acid.

A liquid composition will generally consist of a suspension or solution of the compound or pharmaceutically acceptable salt in a suitable liquid carrier(s). Non-limiting examples include ethanol, glycerine, sorbitol, non-aqueous solvent such as polyethylene glycol, oils or water, with a suspending agent, preservative, surfactant, wetting agent, flavoring or coloring agent. Alternatively, a liquid formulation can be prepared from a reconstitutable powder.

For example, a powder containing active compound, suspending agent, sucrose and a sweetener can be reconstituted with water to form a suspension; and a syrup can be prepared from a powder containing active ingredient, sucrose and a sweetener.

A composition in the form of a tablet can be prepared using any suitable pharmaceutical carrier(s) routinely used for preparing solid compositions. Non-limiting examples of such carriers include magnesium stearate, starch, lactose, sucrose, microcrystalline cellulose and binders, for example, polyvinylpyrrolidone. The tablet can also be provided with a color film coating, or color included as part of the carrier(s). In addition, active compound can be formulated in a controlled release dosage form as a tablet comprising a hydrophilic or hydrophobic matrix.

A composition in the form of a capsule can be prepared using routine encapsulation procedures, for example, by incorporation of active compound and excipients into a hard gelatin capsule. Alternatively, a semi-solid matrix of active compound and high molecular weight polyethylene glycol can be prepared and filled into a hard gelatin capsule; or a solution of active compound in polyethylene glycol or a suspension in edible oil, for example, liquid paraffin or fractionated coconut oil can be prepared and filled into a soft gelatin capsule.

Tablet binders that can be included are acacia, methylcellulose, sodium carboxymethylcellulose, poly-vinylpyrrolidone (Povidone), hydroxypropyl methylcellulose, sucrose, starch and ethylcellulose. Lubricants that can be used include magnesium stearate or other metallic stearates, stearic acid, silicone fluid, talc, waxes, oils and colloidal silica. Flavoring agents such as peppermint, oil ofwintergreen, cherry flavoring or the like can also be used. Additionally, it may be desirable to add a coloring agent to make the dosage form more attractive in appearance or to help identify the product. The compounds and their pharmaceutically acceptable salts that are active when given parenterally can be formulated for intramuscular, intrathecal, or intravenous administration. A typical composition for intramuscular or intrathecal administration will be of a suspension or solution of active ingredient in an oil, for example, arachis oil or sesame oil. A typical composition for intravenous or intrathecal administration will be a sterile isotonic aqueous solution containing, for example, active ingredient and dextrose or sodium chloride, or a mixture of dextrose and sodium chloride. Other examples are lactated Ringer's injection, lactated Ringer's plus dextrose injection, Normosol-M and dextrose, Isolyte E, acylated Ringer's injection, and the like. Optionally, a co-solvent, for example, polyethylene glycol, a chelating agent, for example, ethylenediamine tetraacetic acid, and an anti-oxidant, for example, sodium metabisulphite may be included in the formulation. Alternatively, the solution can be freeze dried and then reconstituted with a suitable solvent just prior to administration. The compounds and their pharmaceutically acceptable salts which are active on rectal administration can be formulated as suppositories. A typical suppository formulation will generally consist of active ingredient with a binding and/or lubricating agent such as a gelatin or cocoa butter or other low melting vegetable or synthetic wax or fat. The compounds and their pharmaceutically acceptable salts which are active on topical administration can be formulated as transdermal compositions or transdermal delivery devices ("patches"). Such compositions include, for example, a backing, active compound reservoir, a control membrane, liner and contact adhesive. Such transdermal patches may be used to provide continuous or discontinuous infusion of the compounds in controlled amounts. The patches may be constructed for continuous, pulsatile, or on demand delivery of pharmaceutical agents. Optionally, the pharmaceutical composition may contain other pharmaceutically acceptable components, such a buffers, surfactants, antioxidants, viscosity modifying agents, preservatives and the like. Other components suitable for use in the formulations can be found in Remington's Pharmaceutical Sciences, Mace Publishing Company, Philadelphia, Pa., 17th ed. (1985).

### Kits

Also disclosed are reagents and kits thereof for practicing one or more of the above-described methods. The subject reagents and kits thereof may vary greatly. Typically, the kits at least include a miRNA agent as described above. The kits may also include a pharmaceutically acceptable delivery vehicle, which may be combined with or separate from the miRNA agent in the kit, e.g., where the two components may be in the same or separate containers in the kit.

In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

### Systems

Also disclosed are systems that find use in practicing the subject methods, as described above. For example, systems for practicing the subject methods may include one or more pharmaceutical formulations, which include the miRNA agent. The term "system" as employed herein refers to a collection of components (e.g., active agent, delivery vehicle, etc, present in a single composition or as disparate compositions) that are brought together for the purpose of practicing the subject methods. For example, separately obtained active agent and delivery vehicle brought together and co-administered to a subject are a system according to the present disclosure.

### References

1. Jemal A, Siegel R, Ward E, et al. Cancer statistics, 2006. CA Cancer J Clin. Mar-Apr 2006;56(2):106-130.
2. Cowgill SM, Muscarella P. The genetics of pancreatic cancer. Am J Surg. Sep 2003;186(3):279-286.
3. Bartel DP. MicroRNAs: genomics, biogenesis, mechanism, and function. Cell. Jan 232004;116(2):281-297.
4. Iorio MV, Ferracin M, Liu CG, et al. MicroRNA gene expression deregulation in human breast cancer. Cancer Res. Aug 15 2005;65(16):7065-7070.
5. Calin GA, Ferracin M, Cimmino A, et al. A MicroRNA signature associated with prognosis and progression in chronic lymphocytic leukemia. N Engl J Med. Oct 27 2005;353(17):1793-1801.
6. Esquela-Kerscher A, Slack FJ. Oncomirs - microRNAs with a role in cancer. Nat Rev Cancer. Apr 2006;6(4):259-269.
7. Volinia S, Calin GA, Liu CG, et al. A microRNA expression signature of human solid tumors defines cancer gene targets. Proc Natl Acad Sci U S A. Feb 14 2006;103(7):2257-2261.
8. Liu CG, Calin GA, Meloon B, et al. An oligonucleotide microchip for genome-wide microRNA profiling in human and mouse tissues. Proc Natl Acad Sci USA. Jun 29 2004;101(26):9740-9744.
9. Tusher VG, Tibshirani R, Chu G. Significance analysis of microarrays applied to the ionizing radiation response. Proc Natl Acad Sci USA. Apr 24 2001;98(9):5116-5121.
10. Tibshirani R, Hastie T, Narasimhan B, Chu G. Diagnosis of multiple cancer types by shrunken centroids of gene expression. Proc Natl Acad Sci USA. May 14 2002;99(10):6567-6572.
11. De Lott LB, Morrison C, Suster S, Cohn DE, Frankel WL. CDX2 is a useful marker of intestinal-type differentiation: a tissue microarray-based study of 629 tumors from various sites. Arch Pathol Lab Med. Sep 2005;129(9):1100-1105.
12. Kulshreshtha R, Ferracin M, Wojcik SE, et al. A MicroRNA Signature of Hypoxia. Mol. Cell. Biol. December 28, 2006 2006:MCB.01395-01306.
13. Chan JA, Krichevsky AM, Kosik KS. MicroRNA-21 is an antiapoptotic factor in human glioblastoma cells. Cancer Res. Jul 15 2005;65(14):6029-6033.
14. Meng F, Henson R, Lang M, et al. Involvement of human micro-RNA in growth and response to chemotherapy in human cholangiocarcinoma cell lines. Gastroenterology. Jun 2006;130(7):2113-2129.
15. Kluiver J, Haralambieva E, de Jong D, et al. Lack of BIC and microRNA miR-155 expression in primary cases of Burkitt lymphoma. Genes Chromosomes Cancer. Feb 2006;45(2):147-153.
16. Kluiver J, Poppema S, de Jong D, et al. BIC and miR-155 are highly expressed in Hodgkin, primary mediastinal and diffuse large B cell lymphomas. J Pathol. Oct 2005;207(2):243-249.
17. Costinean S, Zanesi N, Pekarsky Y, et al. Pre-B cell proliferation and lymphoblastic leukemia/high-grade lymphoma in E(mu)-miR155 transgenic mice. Proc Natl Acad Sci US A. May 2 2006;103(18):7024-7029.
18. Pallante P, Visone R, Ferracin M, et al. MicroRNA deregulation in human thyroid papillary carcinomas. Endocr Relat Cancer. Jun 2006;13(2):497-508.
19. Poliseno L, Tuccoli A, Mariani L, et al. MicroRNAs modulate the angiogenic properties of HUVEC. Blood. Jul 18 2006.
20. Poy MN, Eliasson L, Krutzfeldt J, et al. A pancreatic islet-specific microRNA regulates insulin secretion. Nature. Nov 11 2004;432(7014):226-230.
21. Roldo C, Missiaglia E, Hagan JP, et al. MicroRNA expression abnormalities in pancreatic endocrine and acinar tumors are associated with distinctive pathologic features and clinical behavior. J Clin Oncol. Oct 10 2006;24(29):4677-4684.
22. Bloomston M, Durkin A, Yang I, et al. Identification of molecular markers specific for pancreatic neuroendocrine tumors by genetic profiling of core biopsies. Ann Surg Oncol. Apr 2004;11(4):413-419.
23. Saito Y, Liang G, Egger G, et al. Specific activation of microRNA-127 with downregulation of the proto-oncogene BCL6 by chromatin-modifying drugs in human cancer cells. Cancer Cell. Jun 2006;9(6):435-443.
24. Yekta S, Shih Ih, Bartel DP. MicroRNA-Directed Cleavage of HOXB8 mRNA. Science. April 23, 2004 2004;304(5670):594-596.
25. Mizusawa N, Hasegawa T, Ohigashi I, et al. Differentiation phenotypes of pancreatic islet [beta]- and [alpha]-cells are closely related with homeotic genes and a group of differentially expressed genes. Gene. 2004;331:53-63.
26. Garcea G, Neal CP, Pattenden CJ, Steward WP, Berry DP. Molecular prognostic markers in pancreatic cancer: a systematic review. Eur J Cancer. Oct 2005;4(15):2213-2236.
27. Ghaneh P, Kawesha A, Evans JD, Neoptolemos JP. Molecular prognostic markers in pancreatic cancer. J Hepatobiliary Pancreat Surg. 2002;9(1):1-11.
28. Hezel AF, Kimmelman AC, Stanger BZ, Bardeesy N, Depinho RA. Genetics and biology of pancreatic ductal adenocarcinoma. Genes Dev. May 15 2006;20(10):1218-1249.

## Claims

1. A method of determining the prognosis of a subject with pancreatic cancer, comprising measuring the level of at least miR-196a-2 in a test sample from said subject, wherein a decrease in the level of the at least one miR gene product, relative to the level of the corresponding miR gene product in a control sample, is indicative of an adverse prognosis.

2. The method of claim 1, wherein the level of miR-196a-2 and miR-219 is measured.

3. The method of claim 1, wherein the method of determining the prognosis of a subject with pancreatic cancer comprises determining whether a pancreatic cancer in said subject has a high proliferation index.

4. The method of claim 1, wherein subjects with high miR-196a-2 expression have a median survival of 14.3 months.

5. A method of diagnosing whether a subject has, or is at risk for developing, a pancreatic cancer with an adverse prognosis in a subject, comprising:
(1) reverse transcribing at least miR-196a-2 from a test sample obtained from the subject to provide target oligodeoxynucleotides;
(2) hybridizing the target oligodeoxynucleotides to a microarray comprising miRNA-specific probe oligonucleotides to provide a hybridization profile for said test sample; and
(3) comparing the test sample hybridization profile to a hybridization profile generated from a control sample, wherein a decrease in the level of at least miR-196a-2 is indicative of the subject either having, or being at risk for developing, a pancreatic cancer with an adverse prognosis.

6. The method of claim 5, wherein the level of miR-196a-2 and miR-219 is measured.

7. Use of a kit comprising one or more solid supports having attached thereto one or more oligodeoxynucleotides complementary to miR-196a-2 to determine the prognosis of a subject with pancreatic cancer by measuring the level of at least miR-196a-2 in a test sample from said subject, wherein a decrease in the level of the at least one miR gene product, relative to the level of the corresponding miR gene product in a control sample, is indicative of an adverse prognosis.

8. The use of claim 7, wherein the solid supports have attached thereto one or more oligodeoxynucleotides complementary to miR-196a-2 and miR-219.

9. The use of claim 7 or 8, wherein the solid support is a high-density oligodeoxynucleotide array.

## Patentansprüche

1. Verfahren zum Erstellen der Prognose einer Person mit Pankreaskarzinom, wobei das Verfahren das Messen des Spiegels von mindestens miR-196a-2 in einer Testprobe von der Person umfasst, wobei eine Abnahme des Spiegels des mindestens einen miR-Genprodukts im Vergleich zu dem Spiegel des entsprechenden miR-Genprodukts in einer Kontrollprobe auf eine ungünstige Prognose hindeutet.

2. Verfahren nach Anspruch 1, wobei der Spiegel von miR-196a-2 und miR-219 gemessen wird.

3. Verfahren nach Anspruch 1, wobei das Verfahren zum Erstellen der Prognose einer Person mit Pankreaskarzinom das Bestimmen, ob ein Pankreaskarzinom in der Person einen hohen Proliferationsindex hat, umfasst.

4. Verfahren nach Anspruch 1, wobei Personen mit hoher miR-196a-2-Expression eine mediane Überlebenszeit von 14,3 Monaten haben.

5. Verfahren zum Diagnostizieren, ob eine Person ein Pankreaskarzinom mit einer ungünstigen Prognose in einer Person hat oder ein Risiko zur Entwicklung eines solchen Pankreaskarzinoms hat, wobei das Verfahren Folgendes umfasst:
(1) Umkehrtranskribieren von mindestens miR-196a-2 aus einer Testprobe, die von der Person erhalten wurde, um Ziel-Oligodesoxynukleotide bereitzustellen;
(2) Hybridisieren der Ziel-Oligodesoxynukleotide an ein Mikroarray, das miRNA-spezifische Sonden-Oligonukleotide umfasst, um ein Hybridisierungsprofil für die Testprobe bereitzustellen; und
(3) Vergleichen des Hybridisierungsprofils der Testprobe mit einem Hybridisierungsprofil, das aus einer Kontrollprobe erstellt wurde, wobei eine Abnahme des Spiegels von mindestens miR-196a-2 darauf hindeutet, dass die Person entweder ein Pankreaskarzinom mit einer ungünstigen Prognose hat oder ein Risiko zur Entwicklung eines solchen Pankreaskarzinoms hat.

6. Verfahren nach Anspruch 5, wobei der Spiegel von miR-196a-2 und miR-219 gemessen wird.

7. Verwendung eines Kits, das einen oder mehrere feste Träger umfasst, an denen ein oder mehrere Oligodesoxynukleotide angelagert sind, die zu miR-196a-2 komplementär sind, um die Prognose einer Person mit Pankreaskarzinom zu erstellen, indem der Spiegel von mindestens miR-196a-2 in einer Testprobe von der Person gemessen wird, wobei eine Abnahme des Spiegels des mindestens einen miR-Genprodukts im Vergleich zu dem Spiegel des entsprechenden miR-Genprodukts in einer Kontrollprobe auf eine ungünstige Prognose hindeutet.

8. Verwendung nach Anspruch 7, wobei an den festen Trägern ein oder mehrere Oligodesoxynukleotide angelagert sind, die zu miR-196a-2 und miR-219 komplementär sind.

9. Verwendung nach Anspruch 7 oder 8, wobei der feste Träger ein hochdichtes Oligodesoxynukleotid-Array ist.

## Revendications

1. Procédé de détermination du pronostic d'un sujet atteint d'un cancer du pancréas, consistant à mesurer le taux d'au moins miR-196a-2 dans un échantillon d'essai dudit sujet, une réduction du taux dudit produit génique de miR, par rapport au taux du produit génique de miR correspondant dans un échantillon témoin, est indicative d'un pronostic défavorable.

2. Procédé selon la revendication 1, dans lequel le taux de miR-196a-2 et de miR-219 est mesuré.

3. Procédé selon la revendication 1, dans lequel le procédé de détermination du pronostic d'un sujet atteint d'un cancer du pancréas consiste à déterminer si un cancer du pancréas chez ledit sujet a un indice de prolifération élevé.

4. Procédé selon la revendication 1, dans lequel les sujets présentant une expression élevée de miR-196a-2 ont une survie médiane de 14,3 mois.

5. Procédé pour diagnostiquer si un sujet est atteint, ou est à risque d'un développement d'un cancer du pancréas dont le pronostic est défavorable chez un sujet, consistant à :
(1) effectuer la transcription inverse d'au moins miR-196a-2 d'un échantillon d'essai obtenu chez le sujet pour produire des oligodésoxynucléotides cibles ;
(2) effectuer l'hybridation des oligodésoxynucléotides cibles sur un microréseau comprenant des sondes oligonucléotidiques spécifiques de micro-ARN pour élaborer un profil d'hybridation dudit échantillon d'essai ; et
(3) comparer le profil d'hybridation de l'échantillon d'essai avec un profil d'hybridation généré à partir d'un échantillon témoin, une réduction du taux d'au moins miR-196a-2 indiquant si le sujet est atteint, ou est à risque de développement d'un cancer du pancréas dont le pronostic est défavorable.

6. Procédé selon la revendication 5, dans lequel le taux de miR-196a-2 et de miR-219 est mesuré.

7. Utilisation d'une trousse comprenant un ou plusieurs supports solides auxquels sont attachés un ou plusieurs oligodésoxynucléotides complémentaires de miR-196a-2 pour déterminer le pronostic d'un sujet atteint d'un cancer du pancréas par la mesure du taux d'au moins miR-196a-2 dans un échantillon d'essai dudit sujet, une réduction du taux dudit produit génique de miR, par rapport au taux du produit génique de miR correspondant dans un échantillon témoin, est indicative d'un pronostic défavorable.

8. Utilisation selon la revendication 7, dans laquelle les supports solides comportent, attachés à ces derniers, un ou plusieurs oligodésoxynucléotides complémentaires de miR-196a-2 et de miR-219.

9. Utilisation selon la revendication 7 ou 8, dans laquelle le support solide est un réseau d'oligodésoxynucléotides à haute densité.
